# EUROPEAN PATENT APPLICATION

(11) **EP 4 718 071 A1**
(43) Date of publication of application: **01.04.2026**
(21) Application number: 24810501.7
(22) Date of filing: 25.05.2024
(51) Int. Cl.: G01N 33/66

(54) **MULTI-ANALYTE MONITORING SENSOR**

(30) Priority: 25.05.2023 CN 202310606611
(71) Applicant: Shenzhen Sisensing Co., Ltd., Shenzhen, Guangdong 518000 (CN)
(72) Inventor: CHEN, Liguo, Shenzhen, Guangdong 518000 (CN); FANG, Junfei, Shenzhen, Guangdong 518000 (CN)
(74) Representative: Isern Patentes y Marcas S.L.
(86) International application number: PCT/CN2024/095376
(87) International publication number: WO 2024/240266

(57) **Abstract**

The present disclosure describes a multi-analyte monitoring sensor, which includes a working electrode, an enzyme sensing layer set on the working electrode, and a polymer membrane layer. The working electrode comprises a first working electrode and a second working electrode; the enzyme sensing layer comprises a first analyte enzyme layer and a second analyte enzyme layer, with the first analyte enzyme layer set on the first working electrode and the second analyte enzyme layer set on the second working electrode, and the second analyte being different from the first analyte; the polymer membrane layer is permeable to the first analyte and the second analyte and covers the first analyte enzyme layer and the second analyte enzyme layer. Thus, a multi-analyte monitoring sensor capable of simultaneously monitoring multiple analytes can be provided.

## Description

### BACKGROUND OF INVENTION

### Field of Invention

The present disclosure generally relates to the field of biosensors, and more specifically to a multi-analyte monitoring sensor.

### Description of Related Art

A biosensor is an analytical device that closely integrates biological materials, bio-derived materials, or biomimetic materials with optical, electrochemical, thermal, piezoelectric, magnetic, or micro-mechanical physical and chemical sensors or sensor microsystems. It is usually used to rapidly detect certain specific chemical substances in the human body, such as glucose, ketones, uric acid, and a series of amino acid compounds.

Some substances in the human body may be interrelated. For instance, there is a certain connection between blood ketone levels and blood sugar levels in the human body. When blood sugar is too high, insulin is lacking in the body, or a ketogenic diet is followed, the body will metabolize fat to produce ketones. When the amount of ketones exceeds the liver's metabolic capacity, it is easy to cause complications of diabetes.

Therefore, monitoring different substances in the human body simultaneously can help the host understand their physical condition more conveniently and comprehensively.

### SUMMARY OF INVENTION

This disclosure is made in view of the above situation and aims to provide a multi-analyte monitoring sensor capable of simultaneously monitoring multiple analytes.

Accordingly, the first aspect of the present disclosure provides a multi-analyte monitoring sensor, the sensor comprising a working electrode, an enzyme sensing layer disposed on the working electrode, and a polymer membrane layer. The working electrode includes a first working electrode and a second working electrode; the enzyme sensing layer includes a first analyte enzyme layer and a second analyte enzyme layer, the first analyte enzyme layer being disposed on the first working electrode and the second analyte enzyme layer being disposed on the second working electrode, the second analyte being different from the first analyte; the polymer membrane layer is permeable to the first analyte and the second analyte and covers the first analyte enzyme layer and the second analyte enzyme layer.

In the multi-analyte monitoring sensor involved in this disclosure, by setting enzyme layers of two different analytes on the two working electrodes of the sensor, the concentration levels of two different analytes in the tissue fluid can be detected respectively; the polymer membrane layer can simultaneously permeate the two analytes. By covering the first analyte enzyme layer and the second analyte enzyme layer with the polymer membrane layer, the permeation of the two analytes can be restricted, and at the same time, it is beneficial to improve the stability of the sensor. Additionally, since the biosensor is small in size, if different semi-permeable membranes need to be coated on different analyte electrodes during the sensor processing, the precision requirement of the coating process will be very high. Compared with the scheme of setting different semi-permeable membranes on the first analyte enzyme layer and the second analyte enzyme layer respectively, the polymer membrane layer in this invention can simultaneously permeate the two analytes, thus simplifying the sensor manufacturing process while maintaining the high sensitivity and accuracy of the sensor. Therefore, a sensor with a simple manufacturing process and capable of simultaneously monitoring multiple analytes can be provided.

In the multi-analyte monitoring sensor involved in the first aspect of the present disclosure, optionally, the first analyte is glucose. Thus, glucose can be monitored.

In the multi-analyte monitoring sensor involved in the first aspect of the present disclosure, optionally, the second analyte is any one of β-hydroxybutyrate, lactic acid, ethanol, uric acid, urea, acetylcholine, amylase, bilirubin, cholesterol, chorionic gonadotropin, creatine kinase, creatine, creatinine, DNA, fructosamine, glutamine, hormone, ketones, lactate, oxygen, peroxide, prostate-specific antigen, prothrombin, RNA, troponin, digoxin, digitonin, theophylline, warfarin or antibiotic. Thus, different analytes can be monitored based on different needs.

In the multi-analyte monitoring sensor involved in the first aspect of the present disclosure, optionally, the polymer membrane layer includes a membrane polymer, which is a vinylpyridine-based polymer, polyurethane, sodium polystyrene sulfonate, poly(N-isopropylacrylamide), polyacrylic acid, polyvinyl alcohol, polyvinylpyrrolidone, poly(oligo(ethylene glycol) methyl ether methacrylate), or a block copolymer or blend of the above polymers. Thus, it is conducive to forming a polymer membrane layer that has a restrictive permeation effect on two analytes and is biocompatible. Through the polymer membrane layer, it is helpful to control the amount of the analyte within the linear range of the sensor, thereby improving the sensitivity and accuracy of the sensor.

In the multi-analyte monitoring sensor involved in the first aspect of the present disclosure, optionally, the polymer film layer further includes a crosslinker and a regulator, and the mass fraction of the membrane polymer in the polymer film layer is 80% to 100%. Thus, it is conducive to forming a polymer film layer that has a restrictive permeation effect on two analytes and is biocompatible. Through the polymer film layer, the amount of the analyte can be controlled within the linear range of the sensor, thereby improving the sensitivity and accuracy of the sensor.

In the multi-analyte monitoring sensor involved in the first aspect of the present disclosure, the polymer membrane layer may optionally be biocompatible. Thus, it can help improve the safety performance of the sensor.

In the multi-analyte monitoring sensor involved in the first aspect of the present disclosure, optionally, the polymer membrane layer includes a first membrane layer that is biocompatible and has a diffusion-limiting effect on the first analyte and the second analyte.

In the multi-analyte monitoring sensor involved in the first aspect of the present disclosure, optionally, the polymer membrane layer comprises a first membrane layer that has a diffusion-limiting effect on the first analyte and the second analyte, and a second membrane layer that is biocompatible, with the second membrane layer being further away from the enzyme sensing layer than the first membrane layer.

In the multi-analyte monitoring sensor involved in the first aspect of the present disclosure, optionally, the sensitivity of the first working electrode and the sensitivity of the second working electrode differ by no more than six times. In this case, on the one hand, it can help reduce the current difference between the two working electrodes and reduce the interference of the electric field on the sensor system; on the other hand, if the sensitivities of the two analyte electrodes differ too much, it may lead to a larger reaction amount of the analyte at the high-sensitivity electrode, which affects the reaction between the low-sensitivity electrode and the analyte. Setting the sensitivities of the two electrodes to differ by no more than six times can also suppress this situation from occurring, thereby helping to improve the measurement accuracy of the sensor.

In the multi-analyte monitoring sensor involved in the first aspect of the present disclosure, optionally, the area of the first analyte enzyme layer is greater than or less than the area of the second analyte enzyme layer. Due to the different amounts of the two analytes that can pass through the polymer membrane layer, setting the area of the first analyte enzyme layer to be greater than or less than that of the second analyte enzyme layer enables the selection of an appropriate scheme based on the permeation amounts of the two analytes through the polymer membrane layer. For instance, when the amount of the first analyte that can pass through the polymer membrane layer is less than that of the second analyte, by configuring the area of the first analyte enzyme layer to be larger than that of the second analyte enzyme layer, the contact area between the first analyte enzyme layer and the first analyte can be increased, thereby minimizing the current difference between the two working electrodes as much as possible. This helps to keep the current magnitudes on the two working electrodes at the same level, reduces the interference of the electric field on the sensor system, and further enhances the measurement accuracy of the sensor.

In the multi-analyte monitoring sensor involved in the first aspect of the present disclosure, optionally, the polymer membrane layer includes a negatively charged membrane layer that has a diffusion-limiting effect on the first analyte and the second analyte. In this case, the negatively charged membrane layer has a certain repulsive effect on substances with negative charges, and thus can have a stronger diffusion-limiting effect on negatively charged analytes. When the first analyte and the second analyte are a neutral molecule and a negatively charged molecule respectively (for example, the first analyte is glucose and the second analyte is β-hydroxybutyrate), and the permeable amount of the negatively charged molecule is larger, setting the negatively charged membrane layer can help reduce the difference in permeable amounts between the two analytes, thereby minimizing the current difference between the two working electrodes as much as possible and improving the measurement accuracy of the sensor.

In the multi-analyte monitoring sensor involved in the first aspect of the present disclosure, optionally, the second analyte is β-hydroxybutyrate,and the second analyte enzyme layer includes β-hydroxybutyrate dehydrogenase, diaphorase, coenzyme, and electron mediator. In the second analyte enzyme layer, the mass fraction of β-hydroxybutyrate dehydrogenase is 10% to 35%, the mass fraction of diaphorase is 5% to 40%, the mass fraction of coenzyme is 10% to 45%, and the mass fraction of the electron mediator is 10% to 50%. In this case, the reaction sensitivity of the second analyte enzyme layer to β-hydroxybutyrate can be enhanced. Meanwhile, by setting a large-molecule electron mediator in the enzyme layer, β-hydroxybutyrate dehydrogenase can be covalently bound to the electron mediator, thereby further improving stability.

In the multi-analyte monitoring sensor involved in the first aspect of the present disclosure, optionally, the sensor further comprises a substrate, and the working electrode is disposed on the substrate.

In the multi-analyte monitoring sensor involved in the first aspect of the present disclosure, optionally, the sensor further includes a reference electrode and a counter electrode, with the first working electrode and the second working electrode respectively located on opposite sides of the substrate, and the reference electrode and the counter electrode respectively located on opposite sides of the substrate. In this case, a four-electrode dual-channel detection sensor can be formed. Moreover, setting the four electrodes in pairs on opposite sides of the substrate can improve the utilization rate of the substrate and further reduce the interference of the electric field on the sensor system.

According to the present disclosure, a multi-analyte monitoring sensor can be provided, which is capable of simultaneously monitoring multiple analytes, has a simple manufacturing process, and features high sensitivity and stability.

### BRIEF DESCRIPTION OF THE DRAWINGS

The present disclosure will now be further described in detail with reference to the embodiments illustrated in the attached drawings, wherein:
Figure 1 is a schematic diagram showing a multi-analyte monitoring sensor involved in the embodiments of the present disclosure.
Figure 2A is a front schematic diagram showing a multi-analyte monitoring sensor involved in the present disclosure.
Figure 2B is a schematic diagram of the back side of the multi-analyte monitoring sensor involved in the embodiments of the present disclosure.
Figure 3 is a schematic diagram showing the polymer film layer involved in the embodiment of the present disclosure.
Figure 4 is a response current - test concentration curve diagram of Embodiment 1 of the blood glucose + blood ketone sensor involved in the present disclosure.
Figure 5 is a picture of the monitoring probe of Embodiment 2 of the blood glucose + blood ketone sensor involved in the present disclosure.
Figure 6 is a response current - test time curve diagram of the embodiment 2 of the blood glucose + blood ketone sensor involved in the present disclosure.
Figure 7 is a response current - test concentration curve diagram of Embodiment 2 of the blood glucose + blood ketone sensor involved in the present disclosure.
Figure 8 shows the glucose/sodium β-hydroxybutyrate response current-time curve of Embodiment 2 of the blood glucose + blood ketone sensor involved in the present disclosure.
Figure 9 is a response current - test concentration curve diagram of Embodiment 3 of the blood glucose + blood ketone sensor involved in the present disclosure.
Figure 10 is a picture of the monitoring probe of Embodiment 4 of the blood glucose + blood ketone sensor involved in the present disclosure.
Figure 11 is a picture of the monitoring probe of Embodiment 5 of the blood glucose + blood ketone sensor involved in the present disclosure.
Figure 12 is a picture of the monitoring probe of Embodiment 6 of the blood glucose + blood ketone sensor involved in the present disclosure.
Figure 13 is a picture of the monitoring probe of Embodiment 7 of the blood glucose + blood ketone sensor involved in the present disclosure.
Figure 14 is a picture of the monitoring probe of Comparative Embodiment 1 of the blood glucose + blood ketone sensor involved in the present disclosure.
Figure 15 is a response current - test concentration curve diagram of Comparative Embodiment 1 of the blood glucose + blood ketone sensor involved in the present disclosure.
Figure 16 is a picture of the monitoring probe of Comparative Embodiment 2 of the blood glucose + blood ketone sensor involved in the present disclosure.
Figure 17 is a response current - test time curve diagram of Comparative Embodiment 2 of the blood glucose + blood ketone sensor involved in the present disclosure.
Figure 18 is a picture of the monitoring probe of Comparative Embodiment 3 of the blood glucose + blood ketone sensor involved in the present disclosure.
Figure 19 is a picture showing another perspective of the monitoring probe of Comparative Embodiment 3 of the blood glucose + blood ketone sensor involved in the present disclosure.
Figure 20 is a picture of the monitoring probe of Embodiment 1 of the glucose + lactic acid sensor involved in the present disclosure.
Figure 21 is a response current - test time curve diagram of Embodiment 1 of the glucose + lactic acid sensor involved in the present disclosure.
Figure 22 shows pictures of the monitoring probes of Embodiment 1 and Comparative embodiments 1-2 of the blood ketone sensor involved in the present disclosure.
Figure 23 shows pictures of the monitoring probes coated with the polymer membrane layer of Embodiment 1 and Comparative embodiments 1-2 of the blood ketone sensor involved in the present disclosure.
Figure 24 is a response current - test concentration graph of a linear test for Embodiment 1 of the blood ketone sensor involved in the present disclosure.
Figure 25 is a response current - test concentration curve graph of the linear test of Embodiment 1 of the blood ketone sensor involved in the present disclosure.
Figure 26 is a response current - test concentration curve diagram of the stability test of the embodiment 1 of the blood ketone sensor involved in the present disclosure.
Figure 27 is a response current - test concentration curve graph of the stability test of Comparative Embodiment 1 of the blood ketone sensor involved in the present disclosure.
Figure 28 is a response current - test concentration curve graph of the stability test of Comparative Embodiment 2 of the blood ketone sensor involved in the present disclosure.
Figure 29 is a response current - test concentration graph of the linearity test of Comparative Embodiment 3 of the blood ketone sensor involved in the present disclosure.
Figure 30 is a picture of the working electrode of Comparative Embodiment 4 of the blood ketone sensor involved in the present disclosure.
Figure 31 is a picture of the monitoring probe of the sensor of Comparative Embodiment 4 related to the blood ketone sensor disclosed herein.
Figure 32 is a response current - test time curve diagram of the sensor of Comparative Embodiment 4 of the blood ketone sensor involved in the present disclosure.
Figure 33 is a picture showing the working electrode of the sensor of Comparative Embodiment 5 involved in the present disclosure for blood ketone sensors.
Figure 34 is a current-response vs. test concentration graph of the blood ketone sensor of Comparative Embodiment 5 involved in the present disclosure for the linearity test.

### Explanation of reference numerals in the drawings:

10... Monitoring probe, 11... Substrate, 12... First working electrode, 121... First analyte enzyme layer, 13... Second working electrode, 131... Second analyte enzyme layer, 14... Polymer membrane layer, 141... First membrane layer, 143... Second membrane layer, 15... Reference electrode, 16... Counter electrode.

### DETAILED DESCRIPTION

Hereinafter, with reference to the attached drawings, the preferred embodiments of the present disclosure will be described in detail. In the following description, the same reference numerals are assigned to the same components, and repetitive descriptions will be omitted. Additionally, the attached drawings are merely schematic diagrams, and the size ratios between components or the shapes of the components may differ from the actual ones.

It should be noted that the terms "comprise" and "have" and their variations as used in this disclosure, such as a process, method, system, product or device that comprises or has a series of steps or units, are not limited to those steps or units that are clearly listed, but may include or have other steps or units that are not clearly listed or are inherent to these processes, methods, products or devices.

The subheadings and the like involved in the following description of this disclosure are not intended to limit the content or scope of this disclosure. They merely serve as reading aids. Such subheadings should neither be understood as dividing the content of the article nor should the content under the subheadings be restricted merely to the scope of the subheadings.

The first aspect of the present disclosure relates to a multi-analyte monitoring sensor, which can monitor multiple analytes. In the present disclosure, the multi-analyte monitoring sensor can also be referred to as a "multi-analyte sensor", "dual-analyte sensor", "physiological parameter sensor", or "sensor", "sensing probe", "sensing tip", "implantable probe", etc. In specific sensors for two analytes, the sensor can also be named after the analytes, for example, when the sensor monitors glucose and ketones, the sensor can be called a "glucose and ketone sensor" or a "blood glucose and blood ketone sensor".

The sensors involved in the present disclosure can be used to monitor the physiological parameters of the host. Physiological parameters can be parameters such as glucose, urea, uric acid, ketones and a series of amino acid compounds within the host's body. In the present disclosure, the sensors can also be used to detect analytes within the host's body. Analytes can be chemical substances in body fluids. For example, the analyte can be one or more of glucose, acetylcholine, amylase, bilirubin, cholesterol, chorionic gonadotropin, creatine kinase, creatine, creatinine, DNA, fructosamine, glutamine, hormones, ketones, lactate, oxygen, peroxides, prostate specific antigen, prothrombin, RNA, thyroid stimulating hormone or troponin. Additionally, the analyte can also be a drug in body fluids. For example, the analyte can be digitonin, digoxin, theophylline, warfarin or antibiotics (such as gentamicin, vancomycin, etc.).

The second aspect of the present disclosure relates to a semi-permeable membrane for a multi-analyte monitoring sensor.

The third aspect of the present disclosure relates to a blood ketone sensor.

The fourth aspect of the present disclosure relates to a monitoring device, including a multi-analyte monitoring sensor and an electronic component.

Hereinafter, with reference to the attached drawings, the multi-analyte monitoring sensor involved in the present disclosure will be described.

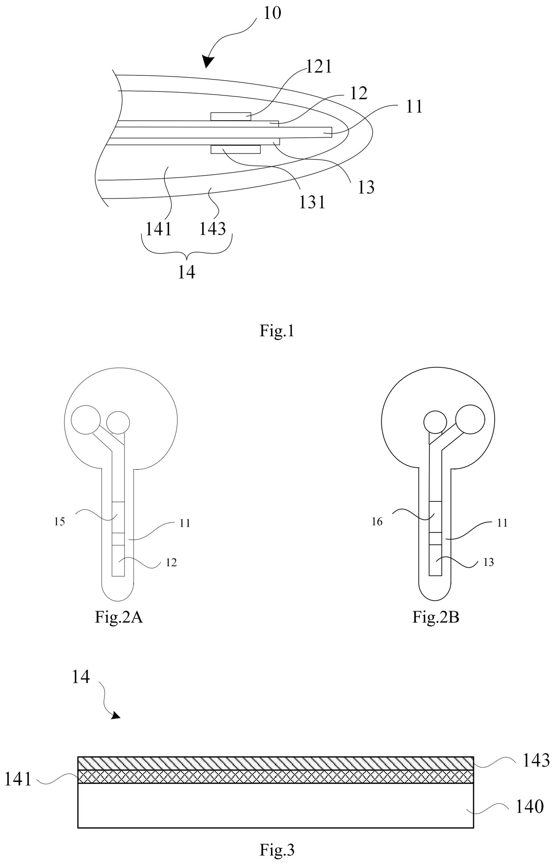

Figure 1 is a schematic diagram showing a multi-analyte monitoring sensor involved in the embodiments of the present disclosure. In Figure 1, for the sake of clearer illustration, the polymer film layer has been magnified. The actual size relationship between the structure of the product and the polymer film layer may not be consistent with the attached figure.

In this embodiment, sensor 10 can be used to obtain physiological parameter information of the host. In some embodiments, sensor 10 can be applied to the host, and the host can obtain their own physiological parameter information through the monitoring device 1 applied to themselves. In some embodiments, sensor 10 can be assembled on the surface of the body of the host, such as the arm, back, abdomen, waist, leg, etc., where sensor 10 can be partially implanted to monitor physiological parameters. In some embodiments, sensor 10 can be used to be implanted under the skin of the host to obtain sensing signals (e.g., current signals). In some embodiments, when sensor 10 is applied to the host, it can come into contact with the tissue fluid or blood inside the host's body to measure the level of analytes in the tissue fluid or blood.

In some embodiments, sensor 10 includes a working electrode. In some embodiments, the number of working electrodes can be multiple, and each working electrode can be used to detect different analytes. Thus, sensor 10 is capable of monitoring multiple analytes.

In some embodiments, the second analyte can be different from the first analyte. Thus, the sensor 10 disclosed herein can detect multiple different analytes. In some embodiments, the working electrode can include a first working electrode 12 and a second working electrode 13, where the first working electrode 12 can be used to detect the first analyte and the second working electrode 13 can be used to detect the second analyte. Thus, the sensor 10 can detect two analytes.

In some embodiments, the first analyte can be any one of glucose, urea, uric acid, ketones, creatinine, ethanol and lactic acid. In some embodiments, the second analyte can be any one of glucose, urea, uric acid, ketones, creatinine, ethanol and lactic acid. Ketone bodies (which can be abbreviated as ketones) are collectively referred to as acetoacetate, β-hydroxybutyrate and acetone, which are intermediate products of fatty acid oxidation in the liver. In the present invention, the content of ketones can be monitored by detecting any one of acetoacetate, β-hydroxybutyrate and acetone. Preferably, the parameter related to the level of ketones can be obtained by monitoring β-hydroxybutyrate in the host body. That is, the second analyte can be β-hydroxybutyrate (which can be abbreviated as β-BHB, β-hydroxybutyric acid or hydroxybutyrate).

In some embodiments, the first analyte can be any one of glucose, β-hydroxybutyrate, lactic acid, ethanol, uric acid, urea, acetylcholine, amylase, bilirubin, cholesterol, chorionic gonadotropin, creatine kinase, creatine, creatinine, DNA, fructosamine, glutamine, hormone, ketones, lactate, oxygen, peroxide, prostate-specific antigen, prothrombin, RNA, troponin, digoxin, digitonin, theophylline, warfarin or antibiotic.

In some embodiments, the second analyte can be any one of glucose, β-hydroxybutyrate, lactic acid, ethanol, uric acid, urea, acetylcholine, amylase, bilirubin, cholesterol, chorionic gonadotropin, creatine kinase, creatine, creatinine, DNA, fructosamine, glutamine, hormones, ketones, lactate, oxygen, peroxides, prostate-specific antigen, prothrombin, RNA, troponin, digoxin, digitonin, theophylline, warfarin or antibiotics.

In some embodiments, the first analyte can be glucose. Thus, the glucose concentration in the body can be monitored. In some embodiments, the second analyte can be ketones. Thus, the ketone content in the body can be monitored. It should be noted that, as mentioned above, ketones are collectively referred to as acetoacetic acid, β-hydroxybutyrate and acetone, which are the intermediate products of fatty acid oxidation in the liver. Therefore, the second analyte can be any one of acetoacetic acid, β-hydroxybutyrate and acetone. Preferably, the second analyte can be β-hydroxybutyrate.

Hereinafter, taking glucose as the first analyte and ketone as the second analyte as an example, a detailed description of the sensor 10 involved in the present disclosure will be provided.

In some embodiments, sensor 10 may include an enzyme sensing layer disposed on the working electrode. In embodiments where the working electrode comprises a first working electrode 12 and a second working electrode 13, the enzyme sensing layer may include a first analyte enzyme layer 121 and a second analyte enzyme layer 131 (see Figure 1). The first analyte enzyme layer 121 may be disposed on the first working electrode 12, and the second analyte enzyme layer 131 may be disposed on the second working electrode 13. In this case, by setting two enzyme layers for different analytes on the two working electrodes of sensor 10, the concentration levels of two different analytes in the tissue fluid can be detected respectively.

In some embodiments, sensor 10 may include a polymer membrane layer 14 (see Figure 1). The polymer membrane layer 14 can be configured to be permeable to the first analyte and the second analyte and cover the first analyte enzyme layer 121 and the second analyte enzyme layer 131. In this case, the polymer membrane layer 14 can limit the permeation of both analytes, while enhancing the stability of the sensor. Additionally, due to the small size of the biosensor, if different semi-permeable membranes need to be coated on different analyte electrodes during the sensor manufacturing process, the precision requirements for the coating process would be very high. Compared to the scheme of setting different semi-permeable membranes on the first analyte enzyme layer 121 and the second analyte enzyme layer 131 respectively, the polymer membrane layer 14 in the present invention can be permeable to both analytes simultaneously, thus simplifying the manufacturing process of the sensor while maintaining its high sensitivity and accuracy. Therefore, a sensor 10 with a simple manufacturing process and the ability to monitor multiple analytes simultaneously can be provided.

In some embodiments, the polymer membrane layer 14 can simultaneously allow the passage of glucose and ketones. In this case, by covering the first analyte enzyme layer 121 and the second analyte enzyme layer 131 with the polymer membrane layer 14, it can restrict the passage of both analytes, and at the same time, it is beneficial for enhancing the stability of the sensor. Additionally, since the biosensor 10 is relatively small in size, the precision requirement for coating the film on the surface of the sensor 10 is relatively high. Usually, the dip-coating method is adopted to cover the sensor 10 with a film solution to form a membrane layer that encloses the sensor 10. Compared with the scheme of separately setting up semi-permeable membranes that allow the passage of glucose and ketones on the first analyte enzyme layer 121 and the second analyte enzyme layer 131, the polymer membrane layer 14 in the present invention can simultaneously allow the passage of glucose and ketones, thereby simplifying the manufacturing process of the sensor 10 while maintaining its high sensitivity and accuracy. Thus, a sensor 10 with a simple manufacturing process and capable of continuously monitoring both glucose and ketones can be provided.

In some embodiments, the permeability coefficient of the polymer membrane layer 14 for the first analyte can be 1 to 1000. In some embodiments, the permeability coefficient of the polymer membrane layer 14 for glucose can be 1 to 1000. Preferably, the permeability coefficient of the polymer membrane layer 14 for glucose can be 300 to 800. For instance, the permeability coefficient of the polymer membrane layer 14 for glucose can be 300, 400, 500, 600, 700 or 800. In some embodiments, the permeability coefficient of the polymer membrane layer 14 for the second analyte can be 1 to 1000. In some embodiments, the permeability coefficient of the polymer membrane layer 14 for ketones can be 1 to 1000. Preferably, the permeability coefficient of the polymer membrane layer 14 for ketones can be 200 to 600. For instance, the permeability coefficient of the polymer membrane layer 14 for ketones can be 200, 300, 400, 500 or 600. Here, the permeability coefficient refers to the concentration difference between the inside and outside of the polymer membrane layer. For example, if the permeability coefficient of the polymer membrane layer 14 for glucose is 500, then the concentration of glucose outside the polymer membrane layer is 500 times that inside the polymer membrane layer.

In some embodiments, the thickness ratio of the polymer film layer 14 can be adjusted to give the polymer film layer 14 different transmission coefficients. In this case, by adjusting the transmission coefficient of the polymer film layer 14, the amount of the analyte passing through can be controlled, making the current generated by the working electrode more accurate and thus making the sensor 10 more sensitive.

Figure 2A is a schematic diagram showing the front of a multi-analyte monitoring sensor involved in the present disclosure. Figure 2B is a schematic diagram of the back side of the multi-analyte monitoring sensor involved in the embodiments of the present disclosure.

In some embodiments, sensor 10 can be used in conjunction with an electronic system, and sensor 10 and the electronic system cooperate to form a sensing component (the sensing component can also be referred to as a monitoring device). Specifically, sensor 10 can be electrically connected to the electronic system, sensor 10 can be partially implanted under the host's skin to obtain a sensing signal indicating the level of the analyte, and then transmit the sensing signal to the electronic system; the electronic system can process and/or retransmit the sensing signal. In this case, the concentration of the analyte in the body can be accurately obtained. Therefore, the present disclosure can also provide a monitoring device, which includes sensor 10 involved in the present disclosure and an electronic system.

In some embodiments, the sensor 10 may include a substrate 11 (see Figure 1). The electrodes of the sensor 10 can be set on the substrate 11.

In some embodiments, sensor 10 may also include a reference electrode 15 and a counter electrode 16 (see Figures 2A and 2B). In this case, a four-electrode dual-channel detection sensor can be formed, that is, the first working electrode 12 can form a three-electrode circuit with the reference electrode 15 and the counter electrode 16, and the second working electrode 13 can also form a three-electrode circuit with the reference electrode 15 and the counter electrode 16.

In some embodiments, the four-electrode dual-channel detection sensor can have a first working electrode 12, a second working electrode 13, a reference electrode 15 and a counter electrode 16, and the first working electrode 12 forms a first circuit with the reference electrode 15 and the counter electrode 16, while the second working electrode 13 forms a second circuit with the reference electrode 15 and the counter electrode 16. In this case, the number of electrodes in the sensor can be reduced, thereby reducing the length of the substrate, and further reducing the implant depth of the sensor 10. Since the greater the implant depth, the more obvious the pain, this can reduce the pain of sensor 10 implantation and improve the wearing comfort.

In some embodiments, the first working electrode 12 and the second working electrode 13 can be respectively located on both sides of the substrate 11, and the reference electrode 15 and the counter electrode 16 can be respectively located on both sides of the substrate 11. In this case, setting the four electrodes in pairs on both sides of the substrate can improve the utilization rate of the substrate, optimize the voltage drop of the sensor electrodes, reduce errors, and further reduce the interference of the electric field on the sensor system. In some embodiments, since the comfort of wearing is related to the depth of implantation, the greater the depth of implantation, the more pain may be experienced during wearing. Therefore, setting the four electrodes in pairs on both sides of the substrate can improve the utilization rate of the substrate, and setting four electrodes in a limited substrate space can reduce the length of the substrate, thereby improving the comfort of wearing.

In some embodiments, the first working electrode 12 and the second working electrode 13 can be located on the same side of the substrate 11, while the reference electrode 15 and the counter electrode 16 can be set on the other side of the substrate 11. In some embodiments, the first working electrode 12, the second working electrode 13 and the reference electrode 15 can be located on the same side of the substrate 11, and the counter electrode 16 can be set on the other side of the substrate 11. In some embodiments, the first working electrode 12, the second working electrode 13 and the reference electrode 15 can be stacked in sequence on the substrate 11. In some embodiments, the electrodes are separated by insulating layers, and the first working electrode 12, the second working electrode 13 and the reference electrode 15 do not completely cover the upper layer electrode.

In some embodiments, the reference electrode 15 can form a known and fixed potential difference with the tissue fluid or blood. In this case, the potential difference between the working electrode and the tissue fluid or blood can be measured by the potential difference formed between the reference electrode 15 and the working electrode. Thus, the voltage generated by the working electrode can be obtained more accurately, and the electronic system can automatically adjust and maintain the stability of the voltage at the working electrode according to the preset voltage value, so that the measured current signal can more accurately reflect the concentration level information of the analyte in the tissue fluid or blood. In some embodiments, the number of reference electrodes 15 can be one or more, for example, two. In this case, the number of reference electrodes can be adjusted according to the number of working electrodes or the current generated by the working electrodes, thereby obtaining an accurate current signal and improving the sensitivity of the sensor.

In some embodiments, sensor 10 can be divided into an implantable part and a connecting part. The implantable part can be placed under the host's skin and connected to the electronic system through the connecting part. In this case, by placing the implantable part under the host's skin and electrically connecting the implantable part to the electronic system through the connecting part, the sensing signals obtained by the implantable part can be conveniently transmitted to the electronic system. In some embodiments, the implantable part can be rigid. This can help it be placed under the host's skin. In other embodiments, the implantable part can also be flexible. In this case, the host's foreign body sensation can be reduced. In some embodiments, a polymer film layer 14 can cover the implantable part of sensor 10. This can help improve the biocompatibility of the implantable part.

In some embodiments, as mentioned above, sensor 10 may include a first analyte enzyme layer 121. In this case, the first analyte can react in the first analyte enzyme layer 121, thereby generating a current and enabling the acquisition of a concentration signal of the first analyte.

In some embodiments, the first analyte enzyme layer 121 can include glucose oxidase. Thus, glucose can react in the first analyte enzyme layer, thereby generating a concentration signal of glucose. In some embodiments, the content (mass fraction) of glucose oxidase in the first analyte enzyme layer 121 can be 40% to 70%. For instance, the content (mass fraction) of glucose oxidase can be 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, or 70%. Thus, it is conducive to the reaction of glucose.

In some embodiments, the first analyte enzyme layer 121 can include an electron mediator. In some embodiments, the electron mediator can be a redox polymer. In some embodiments, the redox polymer can be a metal redox polymer. In some embodiments, the metal redox polymer can be selected from poly(vinylferrocene), quaternized poly(4-vinylpyridine) of ferricyanide, quaternized poly(1-vinylimidazole) of ferricyanide, quaternized poly(4-vinylpyridine) of ferrocyanide, quaternized poly(1-vinylimidazole) of ferrocyanide, osmium 2,2'-bipyridine complex coordinated to poly(1-vinylimidazole), osmium 2,2'-bipyridine complex coordinated to poly(4-vinylpyridine), ruthenium 2,2'-bipyridine complex coordinated to poly(1-vinylimidazole), ruthenium 2,2'-bipyridine complex coordinated to poly(4-vinylpyridine), cobalt 2,2'-bipyridine complex coordinated to poly(1-vinylimidazole), or cobalt 2,2'-bipyridine complex coordinated to poly(4-vinylpyridine), or at least one of them. Thus, the metal redox polymer can participate in redox reactions through covalent bonds, coordination bonds, or ionic bonds.

In some embodiments, the content (mass fraction) of the electron mediator in the first analyte enzyme layer 121 can be 10% to 40%. For instance, the content (mass fraction) of the electron mediator can be 10%, 11%, 12%, 13%, 14%, 15%, 16%, 17%, 18%, 19%, 20%, 21%, 22%, 25%, 26%, 27%, 28%, 29%, 30%, 31%, 32%, 33%, 34%, 35%, 36%, 37%, 38%, 39%, or 40%. Thus, it can promote the reaction of the first analyte in the first analyte enzyme layer.

In some embodiments, the first analyte enzyme layer 121 can include a crosslinker. In some embodiments, the crosslinker can be PEGDGE. In some embodiments, the crosslinker can be PEGDGE400. In some embodiments, the content (mass fraction) of the crosslinker in the first analyte enzyme layer 121 can be 0% to 20%. For example, the content (mass fraction) of the crosslinker can be 0%, 1%, 2%, 3%, 4%, 5%, 6%, 7%, 8%, 9%, 10%, 11%, 12%, 13%, 14%, 15%, 16%, 17%, 18%, 19%, or 20%. Thus, it is convenient for the sensor to monitor for a long time.

In some embodiments, the sensitivity of the first working electrode 12 and the sensitivity of the second working electrode 13 differ by no more than six times. Here, "differ by no more than six times" means that if the sensitivity of the first working electrode 12 is X, the sensitivity of the second working electrode 13 is no less than X/6 and no more than 6X. In this case, on the one hand, it can help reduce the current difference between the two working electrodes and minimize the interference of the electric field on the sensor system; on the other hand, if the sensitivities of the two analyte electrodes differ too much, it may lead to a larger reaction amount of the analyte at the high-sensitivity electrode, which could affect the reaction between the low-sensitivity electrode and the analyte. Setting the sensitivities of the two electrodes to differ by no more than six times can also prevent this situation, thereby helping to improve the measurement accuracy of the sensor 10.

In some embodiments, preferably, the sensitivity of the first working electrode 12 differs from that of the second working electrode 13 by no more than three times. Thus, it can help reduce the current difference between the two working electrodes. In some embodiments, the ratio of the sensitivity of the first working electrode 12 to that of the second working electrode 13 can be 6:1, 5:1, 4:1, 3:1, 2:1, 1:1, 1:2, 1:3, 1:4, 1:5 or 1:6.

In some embodiments, the area of the first analyte enzyme layer 121 can be larger or smaller than that of the second analyte enzyme layer 131. Due to the different amounts of the two analytes that can pass through the polymer membrane layer 14, setting the area of the first analyte enzyme layer 121 to be larger or smaller than that of the second analyte enzyme layer 131 enables the selection of an appropriate scheme based on the permeability of the two analytes through the polymer membrane layer 14. For instance, when the amount of the first analyte that can pass through the polymer membrane layer 14 is less than that of the second analyte, by configuring the area of the first analyte enzyme layer 121 to be larger than that of the second analyte enzyme layer 131, the contact area between the first analyte enzyme layer 121 and the first analyte can be increased, thereby minimizing the current difference between the two working electrodes as much as possible. This helps to keep the current magnitudes on the two working electrodes at the same level, reduces the interference of the electric field on the sensor system, and further enhances the measurement accuracy of the sensor 10.

In some embodiments, the area of the first analyte enzyme layer 121 can be larger than that of the second analyte enzyme layer 131. Since the amounts of glucose and ketone that the polymer membrane layer 14 can permeate are different, and usually the amount of glucose is less than that of ketone, in this case, by configuring the area of the first analyte enzyme layer 121 to be larger than that of the second analyte enzyme layer 131, the contact area between the first analyte enzyme layer 121 and glucose can be increased as much as possible, thereby minimizing the current difference between the two working electrodes, keeping the current magnitudes on the two working electrodes at the same level, reducing the interference of the electric field on the sensor 10 system, and further improving the measurement accuracy of the sensor 10.

In some embodiments, the area ratio of the first analyte enzyme layer 121 to the second analyte enzyme layer 131 can be greater than 1:1. This can help improve the sensitivity of the sensor 10. In some embodiments, the area ratio of the first analyte enzyme layer 121 to the second analyte enzyme layer 131 can be less than 1.5:1. This can enhance the accuracy of the sensor 10. In some embodiments, the area ratio of the first analyte enzyme layer 121 to the second analyte enzyme layer 131 can be from 1:1 to 1.5:1. This can improve the reaction sensitivity of the first analyte and accurately reflect the concentration level of the analyte in the tissue fluid. In some embodiments, the area ratio of the second analyte enzyme layer 131 to the first analyte enzyme layer 121 can be 1:1.5, 1:1.4, 1:1.3, 1:1.2, or 1:1.1. This can more accurately reflect the concentration level of the analyte in the tissue fluid.

In some embodiments, the area of the first analyte enzyme layer 121 can also be equal to the area of the second analyte enzyme layer 131. In this embodiment, if there is a significant difference in sensitivity between the two analyte working electrodes, other methods can be chosen for adjustment. For instance, the composition or structure of the polymer membrane layer 14 can be adjusted to achieve the desired effect (described in detail later).

In some embodiments, the first analyte enzyme layer 121 and/or the second analyte enzyme layer 131 can be in a linear form. This can enhance the utilization rate of the substrate and improve the sensitivity of the sensor. Due to the current processing level and the small size of the sensor, it is difficult to directly apply a uniform linear enzyme layer on the surface of the working electrode with the existing processing accuracy when applying a very small analyte enzyme layer on the working electrode. In some embodiments of the present disclosure, the enzyme layer solution can be applied to the working electrode by drop coating. The droplets of solution applied to the working electrode can diffuse and connect, forming a continuous linear solution. In this case, compared with the multi-point discrete arrangement scheme, a larger area of enzyme layer can be arranged on the limited area of the working electrode, thereby improving the sensitivity of the sensor 10. In some embodiments, when drop coating, the adjacent two drops of solution can be made to intersect, thereby forming a linear analyte enzyme layer on the working electrode in a way that multiple points are connected into a line. In this case, the processing difficulty of the sensor 10 can be reduced by connecting multiple points into a line, thereby forming a linear analyte enzyme layer and improving the utilization rate of the substrate.

In this disclosure, the phrase "the first analyte enzyme layer 121 and/or the second analyte enzyme layer 131 are in a linear shape" at least includes three embodiments: the first analyte enzyme layer 121 is in a linear shape, the second analyte enzyme layer 131 is in a linear shape, and both the first analyte enzyme layer 121 and the second analyte enzyme layer 131 are in a linear shape. In this disclosure, other expressions of "A and/or B" have the same meaning as the above illustration. In this case, by adjusting the area of the analyte enzyme layers, it is beneficial to enhance the sensitivity and accuracy of the sensor 10 when monitoring two analytes.

Figure 3 is a schematic diagram showing the polymer membrane layer 14 involved in the embodiments of the present disclosure. In Figure 3, the schematic of the analyte enzyme layer is omitted.

In some embodiments, the first working electrode 12 can have a base layer 140. Thus, the stability of the sensor 10 can be enhanced. In some embodiments, the base layer 140 is conductive. Thus, the current signal generated by the analyte reaction can be conducted. In some embodiments, the base layer 140 can be made of at least one material selected from gold, glass carbon, graphite, silver, silver chloride, palladium, titanium, and iridium. In this case, the base layer 140 can have good conductivity and can suppress the electrochemical reaction of the base layer 140, thereby improving the stability of the base layer 140.

In some embodiments, the polymer membrane layer 14 can cover the base layer 140 of the working electrode (see Figure 3). Thus, the fabrication process of the membrane layer of the sensor 10 can be simplified, and the biocompatibility of the sensor 10 can be enhanced, which is conducive to the long-term use of the sensor 10.

In some embodiments, the polymer membrane layer 14 can include a membrane polymer. In some embodiments, the membrane polymer can be vinylpyridine-based polymers, polyurethane, sodium polystyrenesulfonate, poly(N-isopropylacrylamide), polyacrylic acid, polyvinyl alcohol, polyvinylpyrrolidone, poly(oligo(ethylene glycol) methyl ether methacrylate), or block copolymers or blends of the above polymers. Thus, it is conducive to forming a polymer membrane layer 14 that restricts the permeation of two analytes and is biocompatible. Through the polymer membrane layer 14, it helps to control the amount of analytes within the linear range of the sensor 10, thereby enhancing the sensitivity and accuracy of the sensor 10.

In some embodiments, the mass fraction of the membrane polymer in the polymer membrane layer 14 can be 80% to 100%. Thus, it is conducive to forming a polymer membrane layer 14 that has a restrictive permeation effect on two analytes and is biocompatible. Through the polymer membrane layer 14, the amount of the analytes can be controlled within the linear range of the sensor 10, thereby improving the sensitivity and accuracy of the sensor 10. In some embodiments, preferably, the polymer membrane layer 14 can include vinylpyridine-based polymers. Thus, it is conducive to forming a polymer membrane layer 14 that has a restrictive permeation effect on glucose and ketones. In some embodiments, preferably, the mass fraction of the vinylpyridine-based polymer in the polymer membrane layer 14 is 80% to 100%. Thus, it is conducive to forming a polymer membrane layer 14 that has a restrictive permeation effect on glucose and ketones and is biocompatible. Through the polymer membrane, the amount of glucose and ketones can be controlled within the linear range of the sensor, improving the sensitivity and accuracy of the sensor. In some embodiments, the mass fraction of the vinylpyridine-based polymer in the polymer membrane layer 14 is 80%, 81%, 82%, 83%, 84%, 85%, 86%, 87%, 88%, 89%, 90%, 91%, 92%, 93%, 94%, 95%, 96%, 97%, 98%, 99%, or 100%. Thus, it is conducive to forming a stable polymer membrane layer 14, and at the same time, it is conducive to enhancing the restrictive permeation effect of the polymer membrane layer 14 on glucose and ketones.

In some embodiments, the polymer film layer 14 can be biocompatible. Thus, it can help improve the safety performance of the sensor 10.

In some embodiments, the polymer film layer 14 may also include a crosslinker and a modifier. In some embodiments, the crosslinker can be polyethylene glycol diglycidyl ether (PEGDGE). Thus, the stability of the polymer film layer 14 can be enhanced, thereby improving the performance of the sensor 10. In some embodiments, the modifier can be a polydimethylsiloxane modifier (PDMS). In this case, the modifier can reduce the surface tension of the overall film layer, which helps to form a uniform film layer and improve the consistency of the sensor 10.

In some embodiments, the polymer membrane layer 14 may include a first membrane layer 141. The first membrane layer 141 may be arranged around the periphery of the enzyme sensing layer. Thus, it can restrict the permeation of the analyte in the tissue fluid, while preventing other interfering substances from passing through, thereby enhancing the reaction efficiency of the enzyme sensing layer and further improving the sensitivity and stability of the sensor. In some embodiments, the first membrane layer 141 may have a diffusion-limiting effect on the first analyte and the second analyte. In the present disclosure, the first membrane layer 141 may also be referred to as a diffusion-limiting layer. In some embodiments, the first membrane layer 141 may be biocompatible.

In some embodiments, the polymer membrane layer 14 may include a second membrane layer 143. The second membrane layer 143 may have biocompatibility. Thus, the occurrence of inflammatory phenomena caused by the implantation of the sensor 10 in the body can be reduced. In some embodiments, the second membrane layer 143 may be arranged on the first membrane layer 141. In this case, a polymer membrane layer 14 with a limiting permeation effect and a biocompatibility effect can be formed, which is conducive to improving the sensitivity and stability of the sensor 10. In some embodiments, the second membrane layer 143 may be further away from the enzyme sensing layer than the first membrane layer 141. That is to say, the second membrane layer 143 may be arranged at the periphery of the first membrane layer 141. In the present disclosure, the second membrane layer 143 may also be referred to as a biocompatible layer.

In some embodiments, the polymer membrane layer 14 can include a negatively charged membrane layer that has a diffusion-limiting effect on the first analyte and the second analyte. In this case, the negatively charged membrane layer has a certain repulsive effect on substances with negative charges, and thus can have a stronger diffusion-limiting effect on negatively charged analytes. When the first analyte and the second analyte are a neutral molecule and a negatively charged molecule respectively, and the permeability of the negatively charged molecule is greater, setting the negatively charged membrane layer can help reduce the difference in permeability between the two analytes, thereby minimizing the current difference between the two working electrodes as much as possible and improving the measurement accuracy of the sensor. For example, when the first analyte is glucose and the second analyte is β-hydroxybutyrate,since the glucose molecule is a neutral molecule and β-hydroxybutyrate is a negatively charged molecule, and the permeability of β-hydroxybutyrate is greater than that of glucose, in this case, setting the negatively charged membrane layer can help reduce the difference in permeability between glucose and β-hydroxybutyrate.

In some embodiments, the polymer film layer 14 may also include a transition layer. The transition layer can be set between the first film layer 141 and the second film layer 143. Thus, a stable polymer film layer 14 can be formed. That is to say, in some embodiments, the polymer film layer 14 may include a first film layer 141 set in sequence on the enzyme sensing layer, a transition layer formed on the first film layer 141, and a second film layer 143 formed on the transition layer and having biocompatibility. Thus, the bonding stability of the polymer film layer 14 can be improved, thereby facilitating the stability of the sensor 10.

In some embodiments, the first membrane layer 141 can include a first type of polymer. In some embodiments, the first type of polymer can include a water-swellable homopolymer. Thus, it can be beneficial to the first membrane layer. In some embodiments, the water-swellable homopolymer can be selected from polyvinylpyrrolidone, polyurethane, poly(2-hydroxyethyl methacrylate), poly(2-hydroxyethyl acrylate), poly(2-vinylpyridine), poly(4-vinylpyridine), poly(2-hydroxyethyl methacrylate), and poly(2-hydroxyethyl acrylate). Thus, it can form a diffusion-controlled performance, making the concentration ratio of the analyte on both sides of the polymer membrane layer fixed, thereby expanding the linear range of the biosensor response.

In some embodiments, the biocompatibility of the first polymer can be enhanced by grafting biocompatible functional groups onto the first polymer. For instance, by grafting biocompatible functional groups onto poly-4-vinylpyridine, the biocompatibility of this material can be improved. Moreover, since the backbone of the first polymer is hydrophobic, it can retain a certain diffusion-limiting effect. By further adjusting the grafting ratio, the first membrane layer 141 can simultaneously possess both diffusion-limiting properties and biocompatibility.

In some embodiments, the first membrane layer 141 can be a negatively charged membrane layer. In some embodiments, the first type of polymer can include negatively charged groups. Thus, it can help to form the first membrane layer 141 carrying negative ions. In some embodiments, the negatively charged groups can be selected from one or more of sulfonic acid groups and carboxyl groups. In other words, the first type of polymer can include sulfonic acid groups and/or carboxyl groups.

In some embodiments, the first type of polymer can be selected from vinylpyridine-based polymers, polyurethane, sodium polystyrene sulfonate, poly(N-isopropylacrylamide), polyacrylic acid, polyvinyl alcohol, polyvinylpyrrolidone, poly(oligo(ethylene glycol) methyl ether methacrylate), or block copolymers or blends of the above polymers. Thus, a first membrane layer 141 that is biocompatible and restricts the permeation of both analytes can be formed. Through the first membrane layer 141, the amount of analytes can be controlled within the linear range of the sensor 10, thereby enhancing the sensitivity and accuracy of the sensor 10. In some embodiments, it is preferred that the first membrane layer 141 includes vinylpyridine-based polymers. Thus, a membrane layer that restricts the permeation of both analytes can be formed.

In some embodiments, the first membrane layer 141 can be formed from the first membrane liquid. Specifically, the first membrane liquid can be coated onto a predetermined position to form the first membrane layer 141. In some embodiments, the concentration of the first type of polymer in the first membrane liquid can be from 1 mg/mL to 150 mg/mL. For instance, the concentration of the first type of polymer in the first membrane liquid can be 1 mg/mL, 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL, 130 mg/mL, 140 mg/mL or 150 mg/mL. In some embodiments, the concentration of the first type of polymer in the first membrane liquid can be from 20 mg/mL to 80 mg/mL. Since the viscosity of the membrane liquid is lower when the concentration of the solute is lower within a certain range, choosing an appropriate concentration can improve the uniformity of the formed membrane layer within a certain range.

In some embodiments, the second membrane layer 143 can include a second type of polymer. In some embodiments, the second type of polymer can include a water-soluble polymer. In some embodiments, the water-soluble polymer can be one selected from polyvinylpyrrolidone, polyvinyl alcohol, polyvinylpyrrolidone, chitosan, carboxymethyl chitosan, chitosan salts, alginic acid, alginates, hyaluronic acid, hyaluronates, cellulose ethers, cellulose esters, polyvinylpyrrolidone, polyacrylamide, polyacrylic acid, polyvinyl alcohol, sodium polystyrene sulfonate, polyethylene glycol, and polyethylene glycol-polypropylene glycol copolymers. Thus, the biocompatibility of the biosensor can be improved.

In some embodiments, preferably, the second film layer 143 may include a copolymer of vinylpyridine and styrene. Thus, it is conducive to improving the film-forming performance of the polymer film layer 14, and further enhancing the sensitivity of the sensor 10.

In some embodiments, the second membrane layer 143 can be formed from a second membrane liquid. Specifically, the first membrane liquid can be coated onto a predetermined position to form the second membrane layer 143. In some embodiments, the concentration of the second type of polymer in the second membrane liquid can be from 1 mg/mL to 150 mg/mL. In some embodiments, for instance, the concentration of the second type of polymer in the second membrane liquid can be 1 mg/mL, 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL, 130 mg/mL, 140 mg/mL or 150 mg/mL. In some embodiments, the concentration of the second type of polymer in the second membrane liquid can be from 20 mg/mL to 80 mg/mL. Since the viscosity of the membrane liquid is lower when the concentration of the solute in the membrane liquid is lower within a certain range, in this case, choosing an appropriate concentration can improve the uniformity of the formed membrane layer within a certain range.

In some embodiments, the transition layer may include a third type of polymer. Here, the third type of polymer is a copolymer formed by a first monomer that is the same as or similar to the first type of polymer and a second monomer that is the same as or similar to the second type of polymer. Thus, the bonding stability and mechanical strength of the polymer film layer 14 can be enhanced, thereby improving the stability of the sensor 10. As a result, a transition layer with enhanced bonding effect to the first film layer 141 and the second film layer 143 can be formed, which is conducive to the formation of the polymer film layer 14 and helps to expand the response linear range of the sensor 10 and improve its biocompatibility.

In some embodiments, the transition layer can include a copolymer of vinylpyridine and styrene. Thus, it is conducive to forming a uniform and stable polymer film layer 14, thereby enhancing the stability of the polymer film layer.

In some embodiments, the analyte enzymes in the first analyte enzyme layer 121 and the second analyte enzyme layer 131 can be selected based on the type of analyte. For instance, when the second analyte is lactic acid, the second analyte enzyme layer 131 can include lactate oxidase. In some embodiments, the analyte enzyme in the second analyte enzyme layer 131 can be 10% to 35%.

In some embodiments, when the second analyte is β-hydroxybutyrate,the second analyte enzyme layer 131 can include β-hydroxybutyrate dehydrogenase. In this case, the β-hydroxybutyrate in the tissue fluid can react under the action of β-hydroxybutyrate dehydrogenase, and the concentration level of β-hydroxybutyrate can be obtained through the signal generated by the reaction, and then the concentration of ketone bodies in the tissue fluid can be mapped based on the concentration level of hydroxybutyric acid. In some embodiments, in the second analyte enzyme layer 131, the content of β-hydroxybutyrate dehydrogenase can be 10% to 30%. For example, the content of hydroxybutyric acid dehydrogenase can be 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, or 35%. Thus, it can help promote the oxidation-reduction reaction of β-hydroxybutyrate in the second analyte enzyme layer 131.

In some embodiments, the second analyte enzyme layer 131 can include albumin. Thus, it can help improve the stability of the second analyte enzyme layer 131. In some embodiments, the second analyte enzyme layer 131 can include human serum albumin.

In some embodiments, the second analyte enzyme layer 131 can include diaphorase. In this case, the electrons generated by the β-BHB reaction can be transferred through diaphorase. In some embodiments, the content of diaphorase in the second analyte enzyme layer 131 can be 5% to 40%. For instance, the content of diaphorase can be 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, or 40%. Thus, it can promote the reaction of the second analyte enzyme layer 131 and simultaneously enhance the electron transfer efficiency in the working electrode.

In some embodiments, the second analyte enzyme layer 131 can include a coenzyme. Thus, it can promote the oxidation of β-hydroxybutyrate dehydrogenase, thereby facilitating the transfer of electrons generated by the β-BHB reaction. In some embodiments, the coenzyme can be nicotinamide adenine dinucleotide (NAD+). In some embodiments, the content of the coenzyme in the second analyte enzyme layer 131 can be 10% to 45%. For instance, the content of the coenzyme can be 10%, 12%, 14%, 15%, 16%, 18%, 19%, 20%, 21%, 22%, 25%, 26%, 27%, 28%, 29%, 30%, 31%, 32%, 33%, 34%, 35%, 36%, 37%, 38%, 39%, 40%, 41%, 42%, 43%, 44%, or 45%. Thus, it can promote the redox reaction between β-hydroxybutyrate dehydrogenase and β-BHB.

In some embodiments, the second analyte enzyme layer 131 can include an electron mediator. In some embodiments, the electron mediator can be a redox polymer. In some embodiments, the redox polymer can be a metal redox polymer. In some embodiments, the metal redox polymer can be selected from at least one of poly(vinylferrocene), quaternized poly(4-vinylpyridine) of ferricyanide, quaternized poly(1-vinylimidazole) of ferricyanide, quaternized poly(4-vinylpyridine) of ferrocyanide, quaternized poly(1-vinylimidazole) of ferrocyanide, osmium 2,2'-bipyridine complex coordinated to poly(1-vinylimidazole), osmium 2,2'-bipyridine complex coordinated to poly(4-vinylpyridine), ruthenium 2,2'-bipyridine complex coordinated to poly(1-vinylimidazole), ruthenium 2,2'-bipyridine complex coordinated to poly(4-vinylpyridine), cobalt 2,2'-bipyridine complex coordinated to poly(1-vinylimidazole), or cobalt 2,2'-bipyridine complex coordinated to poly(4-vinylpyridine). Thus, the metal redox polymer can participate in redox reactions through covalent bonds, coordination bonds, or ionic bonds, and can facilitate the transfer of electrons to the working electrode, thereby generating a current signal corresponding to the concentration of the second analyte.

In some embodiments, the content of the electron mediator in the second analyte enzyme layer 131 can be 10% to 50%. For instance, the content of the electron mediator can be 10%, 12%, 14%, 15%, 16%, 18%, 19%, 20%, 21%, 22%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, or 50%. In this case, the sensitivity of the second analyte enzyme layer 131 to β-BHB can be enhanced. Meanwhile, by setting large-molecule electron mediators in the enzyme layer, the β-hydroxybutyrate dehydrogenase can be covalently bound to the electron mediators, thereby further improving stability.

According to the first aspect of the present disclosure, a multi-analyte monitoring sensor is provided, which is capable of simultaneously and continuously monitoring multiple analytes, has a simple manufacturing process, and features high sensitivity and stability.

The second aspect of the present disclosure relates to a semi-permeable membrane of a multi-analyte monitoring sensor. The semi-permeable membrane of the multi-analyte monitoring sensor involved in the second aspect of the present disclosure is consistent with the polymer membrane layer 14 in the sensor 10 involved in the first aspect of the present disclosure. Regarding the structure of the polymer membrane layer 14, the settings of its various components and parameters, as well as the preparation method, please refer to the description above, and no further elaboration is provided here.

In the second aspect of the present disclosure, a semi-permeable membrane with high stability and capable of simultaneously restricting the permeation of glucose and ketones can be provided. When this semi-permeable membrane is covered on the electrode of the multi-analyte sensor 10, it is beneficial to enhance the sensitivity of the sensor 10.

The third aspect of the present disclosure relates to a blood ketone sensor. The third aspect of the present disclosure relates to a blood ketone sensor which is basically the same as the sensor 10 described in the first aspect of the present disclosure, except that the first working electrode and the first analyte enzyme layer provided thereon are removed from the sensor 10 described in the first aspect, while the second working electrode and the second analyte enzyme layer (i.e., the enzyme layer for detecting ketone) for analyzing ketone are retained. Other parts are the same as those of the sensor 10 described in the first aspect and will not be repeated here. In some embodiments, in the blood ketone sensor, the second working electrode 13 and the reference electrode 15 can be arranged on the same side of the substrate 11, and the counter electrode 16 can be arranged on the other side of the substrate 11. Thus, the utilization rate of the substrate can be improved. Through the blood ketone sensor described in the third aspect of the present disclosure, the ketone level in the host body can be measured.

The fourth aspect of the present disclosure relates to a monitoring device, including the sensor 10 as described in the first aspect of the present disclosure and an electronic system. The electronic system can be electrically connected to the sensor 10. Thus, a monitoring device capable of monitoring multiple analytes can be provided.

Hereinafter, the multi-analyte monitoring sensor and blood ketone sensor provided by the present disclosure will be described in detail in combination with the embodiments and comparative embodiments, but they should not be construed as limiting the scope of protection of the present disclosure.

### [Blood Glucose + Blood Ketone Sensor]

### Embodiment 1

First, prepare a monitoring probe with dual working electrodes at both the front and the back, namely the first working electrode and the second working electrode.

Secondly, prepare the ketone-sensing layer reagent components (osmium-coordinated metal polymer, β-hydroxybutyrate dehydrogenase, diaphorase, NAD+, stabilizer, crosslinker) according to Table 1. Dissolve each substance in the HEPES buffer solvent at the concentration parameters listed in the table to obtain the β-hydroxybutyrate-sensing layer solution.

Subsequently, the glucose-sensing layer solution was prepared with the concentration of osmium-coordinated metal polymer at 10 mg/mL, glucose oxidase (GOD) at 12.5 mg/mL, and crosslinker PEGDGE at 2.5 mg/mL.

The glucose-sensing layer solution was deposited on the first working electrode, and the β-hydroxybutyrate-sensing layer solution was deposited on the second working electrode, both forming a linear pattern. The area of the glucose-sensing layer was approximately 0.27 mm², and the area of the ketone-sensing layer was approximately 0.27 mm²; that is, the ratio of the sensing layer areas for blood ketones and blood glucose was 1:1. After curing, an electrode with a dual-component response sensing layer for glucose and ketone was obtained.

**Table 1. Components of the ketone-sensing layer reagent**

| Substance | Concentration (mg/mL) |
|---|---|
| Metal polymer | 10 |
| β-hydroxybutyrate dehydrogenase | 6 |
| Diaphorase | 6 |
| NAD+ | 6 |
| Human serum albumin | 6 |
| PEGDGE | 5 |

Then, prepare ethanol as the solvent, and mix polyvinylpyridine and PEGDGE according to Table 2 to obtain the diffusion-limiting membrane solution. Apply the diffusion-limiting membrane solution onto the electrode with the sensing layer reagent deposited thereon to fabricate a dual-component monitoring working electrode with a membrane layer of approximately 25 micrometers.

**Table 2. Composition of the diffusion-limiting membrane solution**

| Substance | Concentration (mg/mL) |
|---|---|
| Polyvinylpyridine | 100 |
| Polydimethylsiloxane | 4 |
| PEGDGE | 35 |

Prepare an 80% ethanol aqueous solution. Configure the corresponding concentration solutions of poly(4-vinylpyridine-co-polystyrene) and PEGDGE as per Table 3 to obtain the compatibility membrane solution. Coat the compatibility membrane solution onto the diffusion-limiting membrane of the working electrode to fabricate a working electrode with a compatibility membrane layer of approximately 15 micrometers.

**Table 3. Composition of Compatibility Membrane Solution**

| Substance | Concentration (mg/mL) |
|---|---|
| Poly4 - vinyl pyridine - b - polystyrene | 100 |
| PEGDGE | 15 |

Prepare an 80% ethanol aqueous solution. Configure the corresponding concentration solutions of (Poly4 - vinylpyridine -g- polyethylene glycol) - co - polystyrene and PEGDGE as per Table 4 to obtain the biocompatible membrane solution. Coat the biocompatible membrane solution onto the biocompatible membrane of the working electrode to form a biocompatible membrane layer of approximately 15 microns, resulting in a total membrane thickness of approximately 56 microns for the working electrode.

**Table 4. Components of Biocompatible Membrane Solution**

| Substance | Concentration (mg/mL) |
|---|---|
| (Poly4 - vinylpyridine -g- polyethylene glycol) - co - polystyrene | 100 |
| PEGDGE | 15 |

The sensor from Embodiment 1 was immersed in standard PBS buffer (pH 7.4) at 37°C; a working voltage was applied until a constant background current was reached. The response current was measured simultaneously at the corresponding concentrations of glucose/sodium β-hydroxybutyrate, with each concentration being as follows: 2.2 mM/1 mM, 5 mM/2 mM, 10 mM/3 mM, 15 mM/4 mM, 20 mM/6 mM, 25 mM/8 mM. The former represents the glucose concentration and the latter the sodium β-hydroxybutyrate concentration.

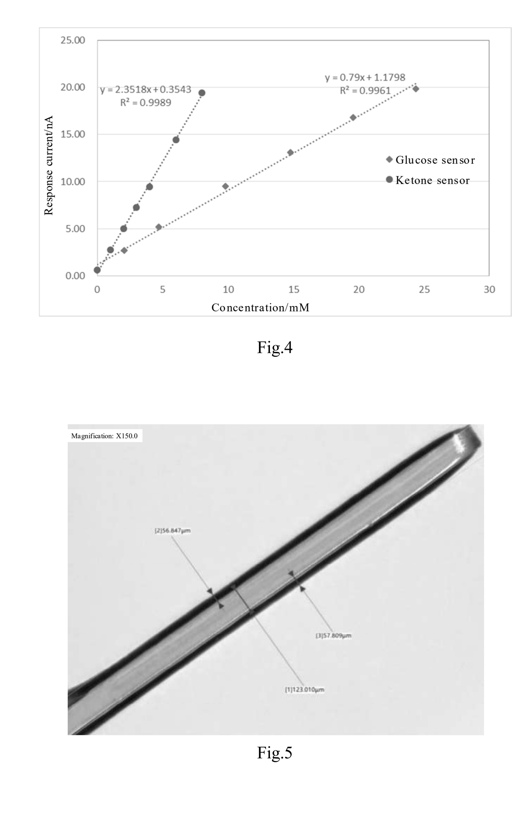

Figure 4 is a response current - test concentration curve diagram of Embodiment 1 of the blood glucose + blood ketone sensor involved in the present disclosure. As shown in Figure 4, the dual-component sensor of Embodiment 1 exhibits excellent linearity in response to glucose and sodium β-hydroxybutyrate. Specifically, the R2 values of the linear current response curves of the sensor to glucose and sodium β-hydroxybutyrate are both greater than 0.996, indicating a good linear relationship. The sensitivity of the glucose working electrode is 0.79 nA/mM, and that of the ketone working electrode is 2.35 nA/mM.

### Embodiment 2

First, prepare a monitoring probe with dual working electrodes at both the front and the back, namely the first working electrode and the second working electrode.

Secondly, the β-hydroxybutyrate-sensing layer solution and the glucose-sensing layer solution were prepared with the same formula as in Embodiment 1, where the metal polymer was a ruthenium coordination metal polymer. The glucose-sensing layer solution was deposited on the first working electrode, and the β-hydroxybutyrate-sensing layer solution was deposited on the second working electrode, both forming a linear pattern, with the glucose sensing layer area being approximately 0.3 mm² and the ketone-sensing layer area being approximately 0.27 mm²; after curing, an electrode containing a dual-component response sensing layer for glucose and ketone was obtained.

The diffusion-limiting membrane, compatibility membrane and biocompatibility membrane were coated in the same manner as in Embodiment 1 to obtain the Embodiment 2 sensor with a ratio of the area of the glucose and ketone sensing layers of 1.11:1.

Figure 5 is a picture of the monitoring probe of Embodiment 2 of the blood glucose + blood ketone sensor involved in the present disclosure.

The sensor from Embodiment 2 was immersed in standard PBS buffer (pH 7.4) at 37 °C. A working voltage was applied until a constant background current was reached. The response current was measured simultaneously for the corresponding concentrations of glucose and sodium β-hydroxybutyrate,with each concentration being as follows: 2.2 mM/1 mM, 5 mM/2 mM, 10 mM/3 mM, 15 mM/4 mM, 20 mM/6 mM, 25 mM/8 mM. The first value represents the glucose concentration and the second value represents the sodium β-hydroxybutyrate concentration.

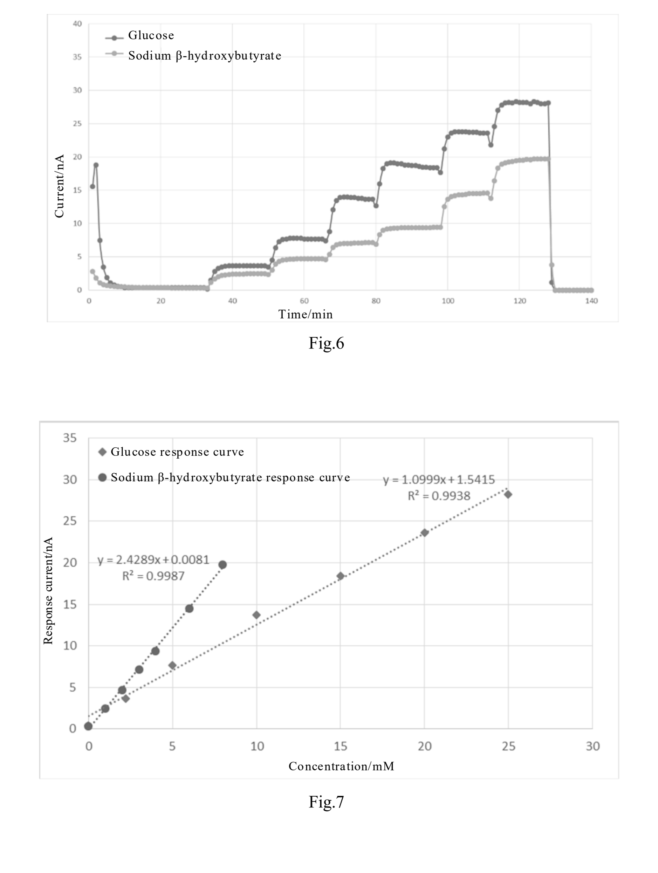

Figure 6 is a response current - test time curve diagram of the embodiment 2 of the blood glucose + blood ketone sensor involved in the present disclosure. Figure 7 is a response current - test concentration curve diagram of the embodiment 2 of the blood glucose + blood ketone sensor involved in the present disclosure. As shown in Figures 6 and 7, the dual-component sensor of embodiment 2 exhibits good linearity in response to glucose and sodium β-hydroxybutyrate. Specifically, as shown in Figure 7, the R2 values of the response current linear curves of the sensor to glucose and sodium β-hydroxybutyrate are both greater than 0.99, indicating a good linear relationship. The sensitivity of the glucose working electrode is 1.10 nA/mM, and the sensitivity of the ketone working electrode is 2.43 nA/mM.

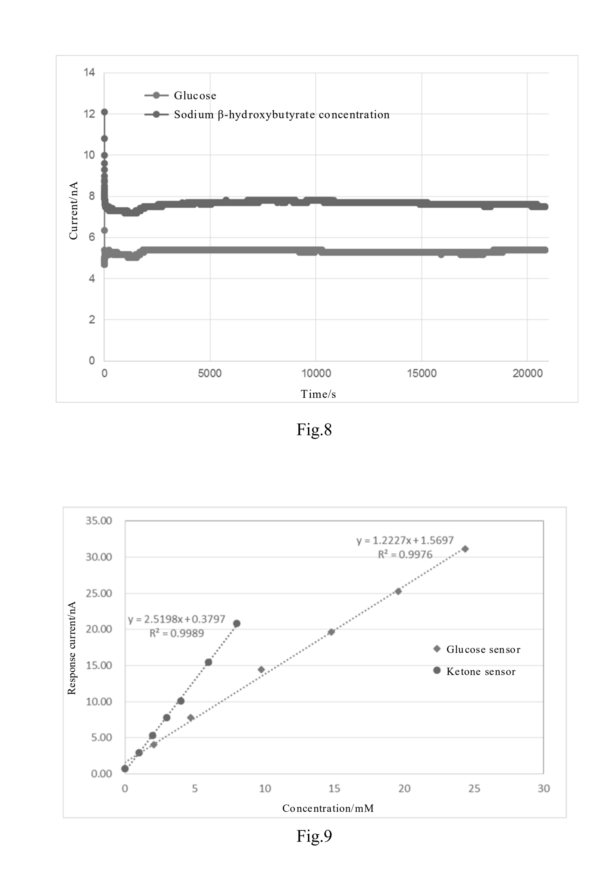

The sensor of Embodiment 2 was immersed in standard PBS buffer (pH 7.4) at 37°C for 336 hours (14 days). Figure 8 shows the glucose/sodium β-hydroxybutyrate response current-time curve of the glucose + blood ketone sensor involved in the present disclosure in Embodiment 2. As shown in Figure 8, during the test period from day 0 to day 14, both current signals of the sensor remained in a stable state.

### Embodiment 3

The monitoring probe with dual working electrodes was prepared in the same way as in Embodiment 1. The glucose-sensing layer solution was deposited on the first working electrode, and the β-hydroxybutyrate-sensing layer solution was deposited on the second working electrode, both forming a linear pattern. The area of the glucose-sensing layer was approximately 0.39 mm², and the area of the ketone-sensing layer was approximately 0.26 mm²; that is, the ratio of the area of the ketone-sensing layer to the glucose-sensing layer was 1:1.5. After curing, the electrode with dual-component response sensing layers for glucose and ketone was obtained.

Then, the diffusion-limiting membrane, compatibility membrane and biocompatibility membrane were coated in the same way as in Embodiment 1 to obtain the Embodiment 3 sensor with a ketone to glucose sensing layer area ratio of 1:1.5.

The sensor from Embodiment 3 was immersed in standard PBS buffer (pH 7.4) at 37 °C; a working voltage was applied until a constant background current was reached. The response current was measured simultaneously at the corresponding concentrations of glucose/sodium β-hydroxybutyrate, with each concentration being as follows: 2.2 mM/1 mM, 5 mM/2 mM, 10 mM/3 mM, 15 mM/4 mM, 20 mM/6 mM, 25 mM/8 mM. The former represents the glucose concentration and the latter represents the sodium β-hydroxybutyrate concentration.

Figure 9 shows the response current - test concentration curve of the glucose + blood ketone sensor of Embodiment 3 involved in the present disclosure. As shown in Figure 9, the dual-component sensor of Embodiment 3 exhibits good linearity in response to glucose and sodium β-hydroxybutyrate. Specifically, the R2 values of the response current linear curves of the sensor to glucose and sodium β-hydroxybutyrate are both greater than 0.997, indicating a good linear relationship. The sensitivity of the glucose working electrode is 1.22 nA/mM, and the sensitivity of the ketone working electrode is 2.52 nA/mM.

### Embodiment 4 - Embodiment 7

First, prepare a monitoring probe with dual working electrodes at both the front and back, namely the first working electrode and the second working electrode.

Secondly, prepare the reagent raw materials for the ketone-sensing layer (osmium-coordinated metal polymer, β-hydroxybutyrate dehydrogenase, diaphorase, NAD+, stabilizer, crosslinker, etc.) according to the table below. Dissolve each substance in the corresponding solvent or buffer solvent at the concentration parameters listed in the table, and ultrasonically shake to ensure complete dissolution, thereby obtaining the β-hydroxybutyrate-sensing layer solutions for Embodiments 4-7.

| Substance | Ketone-sensing Layer |
|---|---|
| Metal polymer | 12 mg/mL |
| β-Hydroxybutyrate dehydrogenase | 8 mg/mL |
| Diaphorase | 8 mg/mL |
| NAD+ | 8 mg/mL |
| Human serum albumin | 8 mg/mL |
| PEGDGE | 4 mg/mL |

Similarly, prepare the glucose-sensing layer solution (osmium-coordinated metal polymer, glucose oxidase, crosslinker, etc.) according to the table below;

| Substance | Glucose-sensing Layer |
|---|---|
| Metal polymer | 12.5 mg/mL |
| Glucose oxidase | 12.5 mg/mL |
| PEGDGE | 2.5 mg/mL |

The approximate glucose-sensing layer solution was deposited on the first working electrode, and the β-hydroxybutyrate-sensing layer solution was deposited on the second working electrode, both forming a linear pattern. The area of the glucose sensing layer was approximately 0.3 mm², and the area of the ketone-sensing layer was approximately 0.3 mm². After curing, a dual-component response sensing layer electrode containing glucose and ketone was obtained.

Then, the electrodes with the modified sensing layer obtained above were coated with the first and second film layers by the dip-coating method according to the table below to obtain the sensors of Embodiments 4 to 7.

| Embodiment | First Membrane Layer (Diffusion-limiting Layer) | | | Second Membrane Layer (Biocompatible Layer) | | Thickness of Membrane |
|---|---|---|---|---|---|---|
| | Component | | Concentration mg/ml | Component | Concentration mg/ml | |
| Embodiment 4 | Solute | Polyvinylpyr idine | 60 | (Poly4 - vinylpyridine -g-polyethylene glycol) - co - polystyrene | 80 | Diffusion-limiting Layer: 35 µm |
| | Additive | Polydimethyl siloxane | 2 | / | / | Biocompatible Layer: 25 µm |
| | Crosslinker | PEGDGE | 10 | PEGDGE | 15 | |
| | Solvent | Ethanol | / | Ethanol | / | |
| Embodiement 5 | Solute | Polyvinylpyr idine | 60 | (Poly4 - vinylpyridine -g-polyethylene glycol) - co - polystyrene | 80 | Diffusion-limiting Layer: 30 µm |
| | Additive | Polydimethyl siloxane | 4 | / | / | Biocompati ble Layer: 25 µm |
| | Crossl inker | PEGDGE | 10 | PEGDGE | 15 | |
| | Solvent | Ethanol | / | Ethanol | / | |
| Embodiment 6 | Solute | (Poly4-vinylpyridine )-co-sodium polystyrene sulfonate | 40 | (Poly4 - vinylpyridine -g-polyethylene glycol) - co - polystyrene | 60 | Diffusion-limiting Layer: 20 µm |
| | Additi ve | / | / | / | / | Biocompatible Layer: 20 µm |
| | Crossl inker | PEGDGE | 7.5 | PEGDGE | 10 | |
| | Solve nt | Ethanol : HEPES = 75 : 25 | / | Ethanol : HEPES = 75 : 25 | / | |
| Embodiment 7 | Solute | (Poly4-vinylpyridine )-co-sodium polystyrene sulfonate | 60 | (Poly4 - vinylpyridine -g-polyethylene glycol) - co - polystyrene | 60 | Diffusion-limiting Layer: 25 µm |
| | Additive | / | / | / | / | Biocompatible Layer: 20 µm |
| | Crossl inker | PEGDGE | 10 | PEGDGE | 10 | |
| | Solvent | Ethanol : HEPES = 75 : 25 | / | Ethanol : HEPES = 75 : 25 | / | |

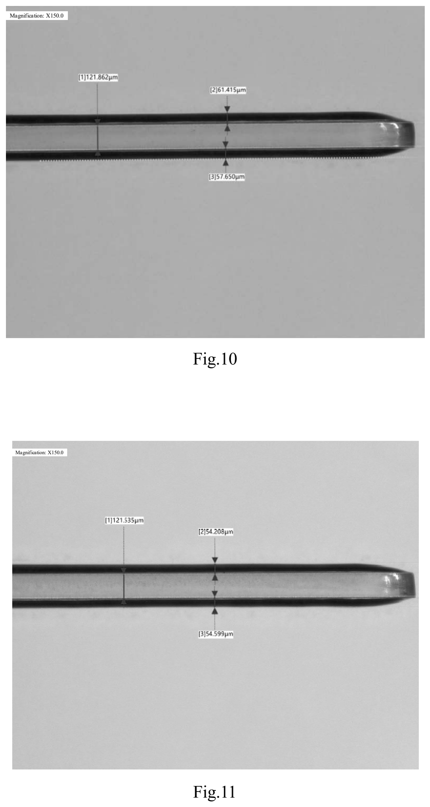

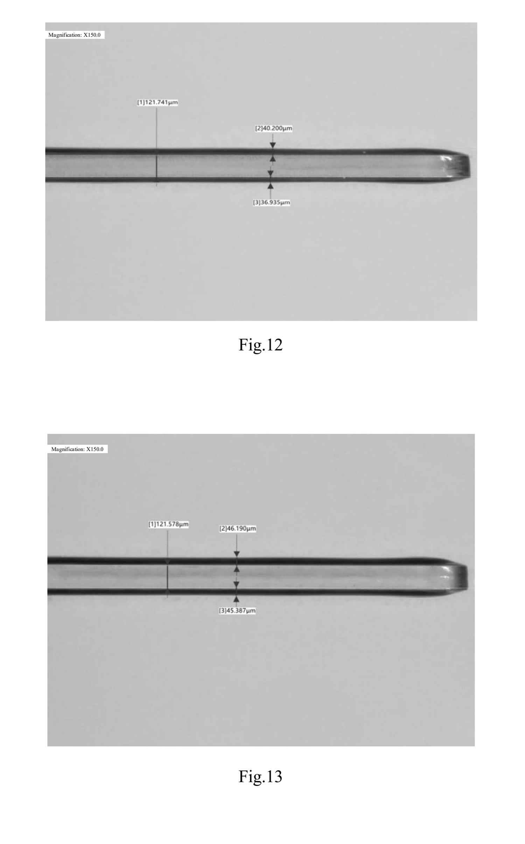

Figures 10 to 13 respectively show the pictures of the monitoring probes of the sensors in Embodiments 4 to 7. It can be seen from Figures 10 to 13 that the morphology of the semi-permeable membranes on each sensor is relatively uniform.

The sensors in Embodiments 4-7 were immersed in standard PBS buffer (pH 7.4) at 37 degrees Celsius. Working voltage was applied until a constant background current was reached. The response currents were measured simultaneously at the corresponding concentrations of glucose/sodium β-hydroxybutyrate. The concentrations were as follows: 2.2 mM/1 mM, 5 mM/2 mM, 10 mM/3 mM, 15 mM/4 mM, 20 mM/6 mM, 25 mM/8 mM, where the former represents the glucose concentration and the latter the sodium β-hydroxybutyrate concentration. The measurement results of each embodiment are shown in the following table:

| Embodiment | Glucose Working Electrode | | | β-Hydroxybutyrate Working Electrode | | |
|---|---|---|---|---|---|---|
| | Sensitivity | Standard Deviation | Linear R² | Sensitivity | Standard Deviation | Linear R² |
| Embodiment 4 | 1.165 | 0.0405 | 0.9964 | 2.561 | 0.0917 | 0.9958 |
| Embodiment 5 | 1.224 | 0.0265 | 0.9951 | 2.881 | 0.0840 | 0.9951 |
| Embodiment 6 | 1.514 | 0.0527 | 0.9937 | 2.247 | 0.0556 | 0.9967 |
| Embodiment 7 | 1.457 | 0.0478 | 0.9949 | 2.078 | 0.0827 | 0.9971 |

Both Embodiment 4 and Embodiment 5 employed polyvinylpyridine as the diffusion-limiting membrane and (Poly4 - vinylpyridine -g- polyethylene glycol) - co - polystyrene as the biocompatible membrane. However, in Embodiment 5, the concentration of PDMS in the diffusion membrane layer was higher, which could reduce the surface tension of the overall membrane layer, making the coating more uniform. At the same time, this would also result in a thinner membrane layer, thus leading to higher sensitivity and a smaller standard deviation.

In embodiments 4 and 5, polyvinylpyridine was used for diffusion limitation. In embodiments 6 and 7, the diffusion membrane was made of poly(4-vinylpyridine-co-sodium styrene sulfonate). Since poly(4-vinylpyridine-co-sodium styrene sulfonate) carries a negative charge, the sensors in embodiments 6 and 7, despite having a thinner membrane layer, showed lower sensitivity to the substrate β-hydroxybutyrate. This is because β-hydroxybutyrate also carries a negative charge in the test solution, thus the membrane material has a certain repulsive effect on it (stronger diffusion limitation effect). However, glucose substrate is a neutral molecule without charge, so the glucose response in embodiments 6 and 7 was higher than that in embodiments 4 and 5.

### Comparative Embodiment 1

First, prepare a monitoring probe with dual working electrodes at both the front and back, namely the first working electrode and the second working electrode.

Secondly, the β-hydroxybutyrate-sensing layer solution and the glucose sensitive layer solution were prepared with the same formula as in Embodiment 1. The glucose sensitive layer solution was deposited on the first working electrode, and the β-hydroxybutyrate-sensing layer solution was deposited on the second working electrode, both forming a linear pattern. The area of the glucose sensing layer was approximately 0.3 mm², and the area of the ketone-sensing layer was approximately 0.45 mm², that is, the area ratio of the glucose enzyme layer to the ketone enzyme layer was 1:1.5. After curing, an electrode with a dual-component response sensing layer for glucose and ketone was obtained.

Then, the above-obtained dual-component response electrode was coated with the three membrane layer formulations in the same manner as in Embodiment 1, and a dual-component sensor with a final membrane layer thickness of approximately 57 micrometers was ultimately obtained.

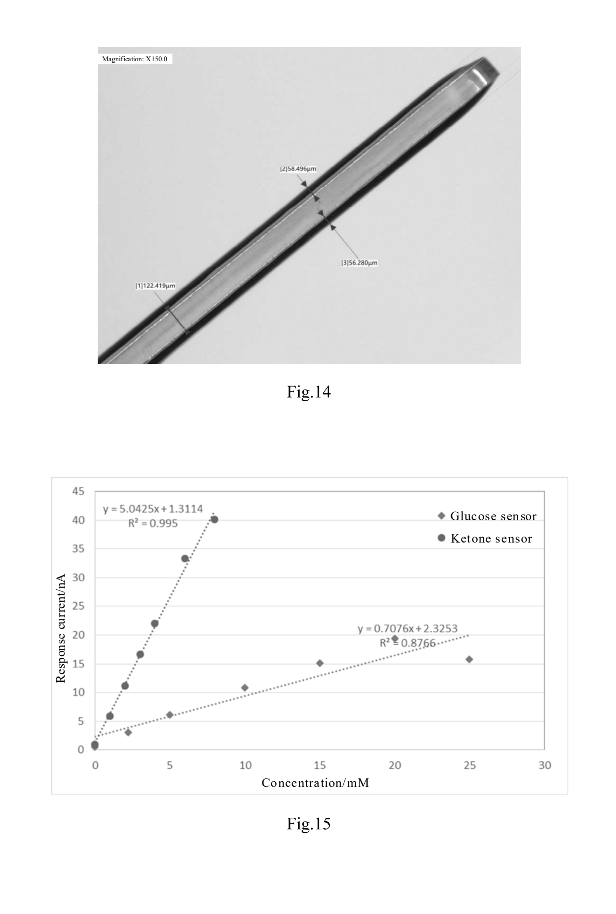

Figure 14 is a picture of the monitoring probe of Comparative Embodiment 1 of the blood glucose + blood ketone sensor involved in the present disclosure.

The sensor in Embodiment 1 was immersed in standard PBS buffer (pH 7.4) at 37°C. A working voltage was applied relative to the reference electrode until a constant background current was reached. The response current was measured simultaneously at the corresponding concentrations of glucose/sodium β-hydroxybutyrate, with the concentrations being 2.2 mM/1 mM, 5 mM/2 mM, 10 mM/3 mM, 15 mM/4 mM, 20 mM/6 mM, and 25 mM/8 mM, respectively, where the former represents the glucose concentration and the latter represents the sodium β-hydroxybutyrate concentration.

Figure 15 is a response current - test concentration curve diagram of Comparative Embodiment 1 of the blood glucose + blood ketone sensor involved in the present disclosure.

The current response of the dual-component sensor in Embodiment 1 to sodium β-hydroxybutyrate concentration shows good linearity, while the current response to glucose concentration is poor. Specifically, as shown in Figure 15, the R2 values of the linear curves of the sensor's response to glucose and sodium β-hydroxybutyrate are 0.877 and 0.995, respectively. This indicates that the glucose sensor does not exhibit linearity within the 0-25 mM range. This may be due to the fact that when responding to high concentrations, the reaction amount on the ketone side is large, and the products affect the response of the working electrode on the glucose side, thereby causing the non-linearity of the glucose sensor at high concentrations.

### Comparative Embodiment 2

First, prepare a monitoring probe with dual working electrodes at both the front and back, namely the first working electrode and the second working electrode.

Secondly, the β-hydroxybutyrate-sensing layer solution and the glucose sensitive layer solution were prepared with the same formula as in Embodiment 1. The glucose sensitive layer solution was deposited on the first working electrode, and the β-hydroxybutyrate-sensing layer solution was deposited on the second working electrode, both forming a linear pattern. The area of the glucose sensing layer was approximately 0.3 mm², and the area of the ketone-sensing layer was approximately 0.27 mm². After curing, an electrode with a dual-component response sensing layer for glucose and ketone obtained.

Then, prepare an 80% ethanol aqueous solution as the solvent. Mix polyvinylpyridine-b-polystyrene and PEGDGE in the corresponding concentrations of ethanol solution as shown in Table 5 to obtain the membrane solution. Apply the membrane solution onto the electrode with the sensing layer reagent deposited to form a dual-component monitoring working electrode with a membrane layer of approximately 60 micrometers.

**Table 5. Composition of Diffusion Limiting Membrane Solution**

| Substance | Concentration (mg/mL) |
|---|---|
| Polyvinyl pyridine | 100 |
| Polydimethylsiloxane | 4 |
| PEGDGE | 35 |

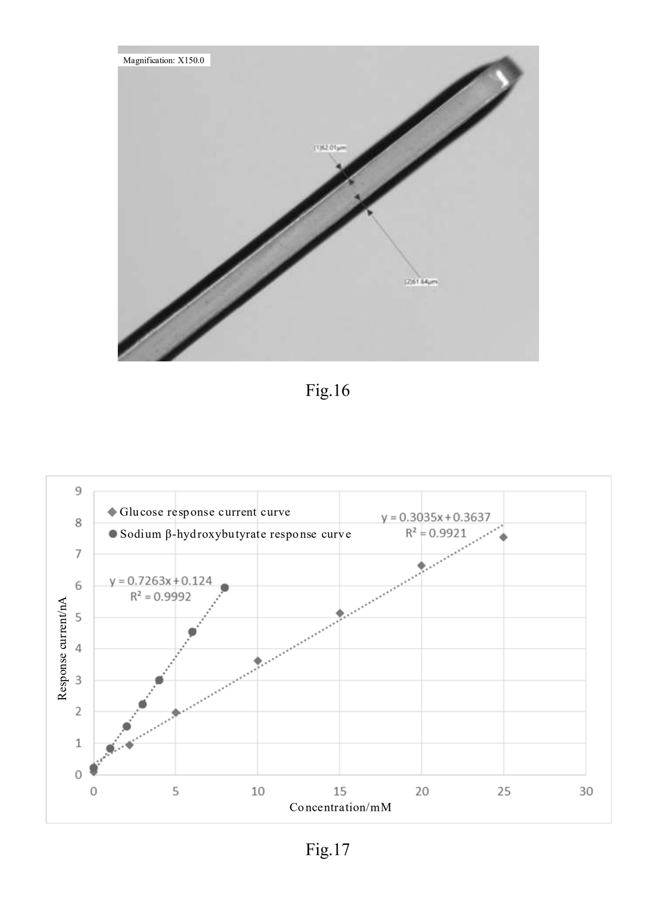

Figure 16 is a picture of the monitoring probe of Comparative Embodiment 2 of the blood glucose + blood ketone sensor involved in the present disclosure.

The sensor in Embodiment 2 was immersed in standard PBS buffer (pH 7.4) at 37°C; the working voltage was applied until a constant background current was reached. The response current was measured simultaneously at the corresponding concentrations of glucose/sodium β-hydroxybutyrate. The concentrations were as follows: 2.2 mM/1 mM, 5 mM/2 mM, 10 mM/3 mM, 15 mM/4 mM, 20 mM/6 mM, 25 mM/8 mM, with the former being the glucose concentration and the latter being the sodium β-hydroxybutyrate concentration.

Figure 17 shows the response current - test time curve of the comparative Embodiment 2 of the blood glucose + blood ketone sensor involved in the present disclosure. As shown in Figure 17, the dual-component sensor of comparative Embodiment 2 shows good linearity in response to glucose and sodium β-hydroxybutyrate. Specifically, the R2 values of the linear curves of the sensor's response current to glucose and sodium β-hydroxybutyrate are both greater than 0.99, indicating a good linear relationship. Among them, the sensitivity of the glucose working electrode is 0.30 nA/mM, and the sensitivity of the ketone working electrode is 0.73 nA/mM. However, compared with Embodiment 2, the sensitivities of the two electrodes in comparative Embodiment 2 are lower, indicating that adding the compatibility membrane layer and the biocompatibility membrane layer can significantly improve the sensitivity of the electrodes.

### Comparative Embodiment 3

First, prepare a monitoring probe with dual working electrodes at both the front and back, namely the first working electrode and the second working electrode.

Secondly, the β-hydroxybutyrate-sensing layer solution and the glucose-sensing layer solution were prepared with the same formula as in Embodiment 1. The glucose-sensing layer solution was deposited on the first working electrode, and the β-hydroxybutyrate-sensing layer solution was deposited on the second working electrode, both forming a linear pattern, where the area of the glucose sensing layer was approximately 0.3 mm² and the area of the ketone-sensing layer was approximately 0.27 mm²; then, they were cured to obtain an electrode with a dual-component response sensing layer for glucose and ketone.

Then, prepare ethanol as the solvent, and configure the corresponding concentration alcohol solution of polyvinylpyridine and PEGDGE according to Table 6 to obtain the diffusion-limiting membrane solution. Apply the diffusion-limiting membrane solution onto the electrode with the sensing layer reagent deposited thereon to make a two-component monitoring working electrode with a membrane layer of approximately 35 micrometers.

**Table 6. Composition of Diffusion Limiting Membrane Solution**

| Substance | Concentration (mg/mL) |
|---|---|
| Polyvinyl pyridine | 100 |
| Polydimethylsiloxane | 4 |
| PEGDGE | 35 |

Finally, prepare an 80% ethanol aqueous solution. Configure the corresponding concentration solutions of (Poly4 - vinylpyridine -g- polyethylene glycol) - co - polystyrene and PEGDGE as per Table 7 to obtain the biocompatible membrane solution. Coat the biocompatible membrane solution onto the diffusion-limiting membrane of the working electrode to form a biocompatible membrane layer of approximately 25 micrometers, resulting in a total membrane thickness of approximately 60 micrometers for the working electrode.

**Table 7. Components of Biocompatible Membrane Solution**

| Substance | Concentration (mg/mL) |
|---|---|
| (Poly4 - vinylpyridine -g- polyethylene glycol) - co - polystyrene | 100 |
| PEGDGE | 15 |

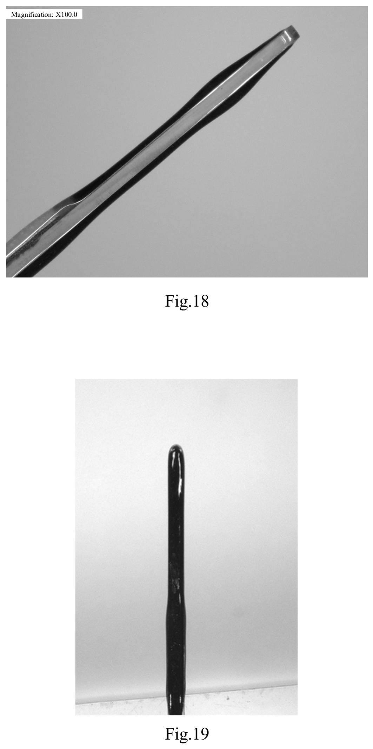

Figure 18 is a picture of the monitoring probe of Comparative Embodiment 3 of the blood glucose + blood ketone sensor involved in the present disclosure. Figure 19 is a picture of the monitoring probe of Comparative Embodiment 3 of the blood glucose + blood ketone sensor involved in the present disclosure from another perspective.

As shown in Figures 18 and 19, it can be seen that when the concentration of the polymer (solute) in the diffusion-limiting membrane solution and the biocompatible membrane solution is relatively high, if there is no compatible membrane layer, the morphology of the semi-permeable membrane of the sensor obtained may become wrinkled, with uneven thickness from top to bottom, which is not conducive to the preparation of highly consistent sensors.

### [Glucose + Lactate Sensor]

### Embodiment 1

First, prepare a monitoring probe with dual working electrodes at both the front and back, namely the first working electrode and the second working electrode.

Secondly, prepare the raw materials for the lactate-sensing layer reagent (osmium-coordinated metal polymer, lactate oxidase, stabilizer, crosslinker, etc.) according to the table below. Dissolve each substance in the corresponding solvent or buffer solution at the concentration parameters indicated in the table. Ultrasonically shake to ensure complete dissolution, and obtain the lactate-sensing layer solution for each embodiment.

| Substance | Lactate-sensing Layer |
|---|---|
| Metal polymer | 20 mg/mL |
| Lactate oxidase | 24.6 mg/mL |
| Human serum albumin | 24.6 mg/mL |
| PEGDGE | 6.2 mg/mL |
| Solvent | HEPES buffer |

Prepare the glucose-sensing layer solution according to the following table as well;

| Substance | Glucose-sensing Layer |
|---|---|
| Metal polymer | 10 mg/mL |
| Glucose oxidase | 12.5 mg/mL |
| PEGDGE | 2.5 mg/mL |
| Solvent | HEPES buffer |

The glucose-sensing layer solution was deposited on the first working electrode and the lactate-sensing layer solution was deposited on the second working electrode, both forming a linear pattern. The area of the glucose sensing layer was approximately 0.3 mm² and that of the lactate sensing layer was approximately 0.30 mm². After curing, an electrode with a dual-component response sensing layer for glucose and lactate was obtained.

Then, ethanol was prepared as the solvent, and polyvinylpyridine and PEGDGE were configured into alcohol solutions of corresponding concentrations as per the table below to obtain the diffusion-limiting membrane solution. A sensor containing a diffusion-limiting layer of approximately 30 micrometers was fabricated by the dip-coating method.

| Substance | Concentration |
|---|---|
| Polyvinyl pyridine | 100 mg/mL |
| Polydimethylsiloxane | 2 mg/mL |
| PEGDGE | 20 mg/mL |

Prepare 80% ethanol aqueous solution as well. Configure the corresponding concentration solutions of poly(4-vinylpyridine-co-polystyrene) and PEGDGE as per the table below to obtain the compatibility membrane solution. The sensor with a compatibility membrane layer of approximately 20 microns is fabricated through the dip-coating method.

| Substance | Concentration |
|---|---|
| Poly4 - vinylpyridine -b-polystyrene | 100 mg/mL |
| PEGDGE | 20 mg/mL |

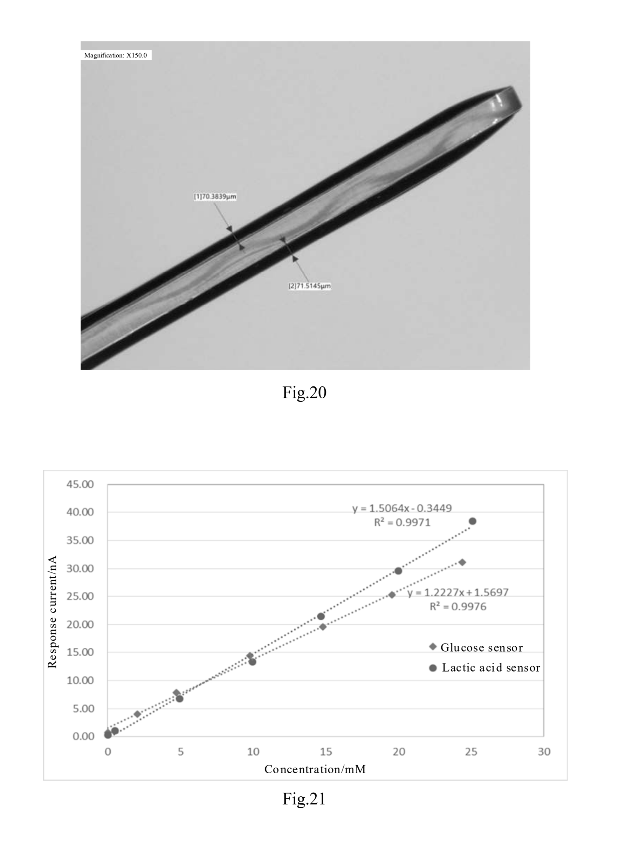

Prepare 80% ethanol aqueous solution as well. Mix (Poly4 - vinylpyridine - g- polyethylene glycol) - co - polystyrene and PEGDGE in accordance with the concentrations listed in the table below to obtain the biocompatible membrane solution. A sensor with a biocompatible membrane layer of approximately 20 microns and a total membrane thickness of approximately 70 microns is fabricated by the dip-coating method. Figure 20 shows a picture of the monitoring probe of Embodiment 1 of the glucose + lactate sensor involved in the present disclosure.

| Substance | Concentration |
|---|---|
| (Poly4 - vinylpyridine -g- polyethylene glycol) - co - polystyrene | 100 mg/mL |
| PEGDGE | 20 mg/mL |

The sensor was immersed in standard PBS buffer (pH 7.4) at 37 degrees. The working voltage was applied until a constant background current was reached. The response currents at various concentrations of glucose and lactate were measured, with the concentrations being 2.2 mM/0.5 mM, 5 mM/5 mM, 10 mM/10 mM, 15 mM/15 mM, 20 mM/20 mM, and 25 mM/25 mM, respectively, where the first value represents the glucose concentration and the second value represents the lactate concentration. Figure 21 shows the response current - test time curve of the glucose + lactate sensor of Embodiment 1 involved in the present disclosure.

As can be seen from Figure 21, the dual-component sensor for glucose and lactate shows excellent linearity in response to both glucose and lactate. The R2 values of the linear curves of the sensor's response currents to glucose and lactate are both greater than 0.997, indicating a good linear relationship. The sensitivity of the glucose working electrode is 1.22 nA/mM, and that of the lactate working electrode is 1.51 nA/mM.

### [Blood Ketone Sensor]

### Embodiment 1, Comparative embodiments 1-2

First, prepare the monitoring probe with the working electrode.

Secondly, based on Table 8, prepare the reagent raw materials for the sensing layers of each blood ketone sensor embodiment and comparative embodiment (ruthenium coordination metal polymer, β-hydroxybutyrate dehydrogenase, diaphorase, NAD+, stabilizer, crosslinker, etc.). Dissolve each substance in the corresponding solvent and buffer solvent according to the concentration parameters in the table, and ultrasonically shake to ensure complete dissolution, thereby obtaining the β-hydroxybutyrate-sensing layer solution for each embodiment.

The β-hydroxybutyrate-sensing layer solution was deposited on the working electrode to form a linear pattern, which was approximately 160 micrometers wide and 2.1 mm long; it was then cured to obtain the enzyme sensing layer.

**Table 8. embodiments of Blood Ketone Sensors**

| Substance | Embodiment 1 | Comparative Example 1 | Comparative Example 2 |
|---|---|---|---|
| Metal polymer (mg/mL) | 10 | 10 | 10 |
| β-Hydroxybutyrate dehydrogenase (mg/mL) | 6 | 6 | 6 |
| Diaphorase (mg/mL) | 6 | 6 | 6 |
| NAD+ (mg/mL) | 6 | 6 | 6 |
| Human serum albumin (mg/mL) | 6 | / | 6 |
| PEGDGE (mg/mL) | 5 | 5 | 5 |
| Solvent | HEPES buffer | HEPES buffer | PBS |

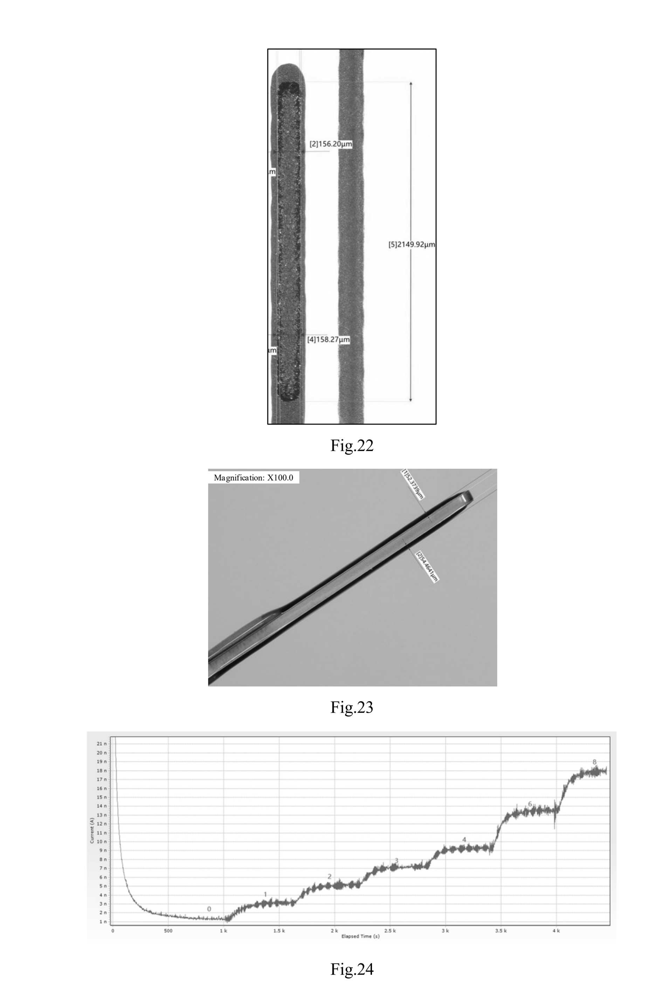

Figure 22 shows pictures of the monitoring probes of Embodiment 1 and Comparative embodiments 1-2 of the blood ketone sensor involved in the present disclosure.

Then, prepare pure ethanol as the solvent, and configure the corresponding concentration ethanol solutions of polyvinylpyridine, polydimethylsiloxane, and PEGDGE as per Table 9 to obtain the diffusion-limiting membrane solution. Apply the diffusion-limiting membrane solution onto the working electrode coated with the β-hydroxybutyrate-sensing layer solution to fabricate a working electrode with a diffusion-limiting membrane layer of approximately 20 micrometers.

**Table 9. Composition of Diffusion Limiting Membrane Solution**

| Substance | Concentration (mg/mL) |
|---|---|
| Polyvinyl pyridine | 92 |
| Polydimethylsiloxane | 3 |
| PEGDGE | 30 |

Prepare an 80% ethanol aqueous solution. Configure the corresponding concentration solutions of poly(4-vinylpyridine-co-polystyrene) and PEGDGE as per Table 10 to obtain the compatibility membrane solution. Coat the working electrode with the diffusion-limiting layer membrane with the compatibility membrane solution to make a working electrode with a compatibility membrane layer of approximately 15 micrometers.

**Table 10. Compatibility Membrane Solution Components**

| Substance | Concentration (mg/mL) |
|---|---|
| Poly4 - vinylpyridine -b-polystyrene | 70 |
| PEGDGE | 10 |

Prepare an 80% ethanol aqueous solution. Configure the corresponding concentration solutions of (Poly4 - vinylpyridine -g- polyethylene glycol) - co - polystyrene and PEGDGE as per Table 11 to obtain the biocompatible membrane solution. Coat the working electrode with the compatibility film layer with the biocompatible membrane solution to make a working electrode with a biocompatible film layer of approximately 15 microns and a total film thickness of 50 microns.

**Table 11. Components of Biocompatible Membrane Solution**

| Substance | Concentration (mg/mL) |
|---|---|
| (Poly4 - vinylpyridine -g- polyethylene glycol) - co - polystyrene | 70 |
| PEGDGE | 10 |

Figure 23 shows pictures of the monitoring probes coated with the polymer membrane layer of Embodiment 1 and Comparative embodiments 1-2 of the blood ketone sensor involved in the present disclosure.

Final test implementation Embodiment 1: Linearity and stability tests of the ketone biosensors obtained in comparative embodiments 1-2:

### (1)Linear test:

The ketone biosensor was immersed in a standard PBS buffer (pH 7.4) at 37°C; a working voltage was applied until the ketone biosensor reached a constant background current. Then, 0 mM, 1 mM, 2 mM, 3 mM, 4 mM, 6 mM, and 8 mM concentrations of sodium β-hydroxybutyrate were added to the solution to measure the reaction linearity. After each addition of sodium β-hydroxybutyrate,the solution was allowed to equilibrate for 10 minutes, and the solution was continuously stirred during the measurement process to ensure uniform concentration.

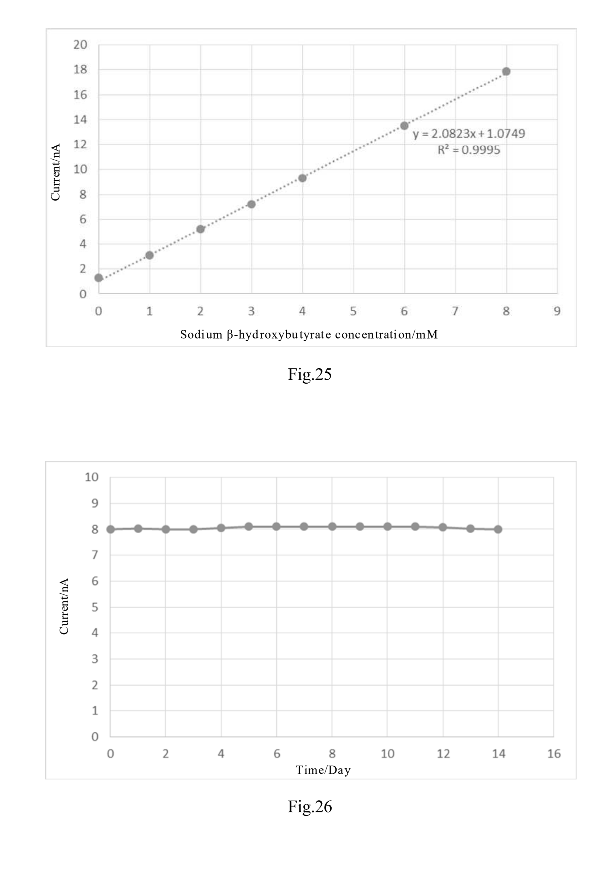

Figure 24 shows the response current - test concentration graph of the linear test of the blood ketone sensor of Embodiment 1 involved in the present disclosure. Figure 25 shows the response current - test concentration curve of the linear test of the blood ketone sensor of Embodiment 1 involved in the present disclosure. As shown in Figures 24 and 25, the ketone biosensor of the blood ketone sensor of Embodiment 1 shows good linearity in response to glucose. Specifically, the glucose sensor in Embodiment 1 has a good linear correlation between the sodium β-hydroxybutyrate concentration and the response current in the range of 0 mM to 8 mM.

### (2) Stability Test:

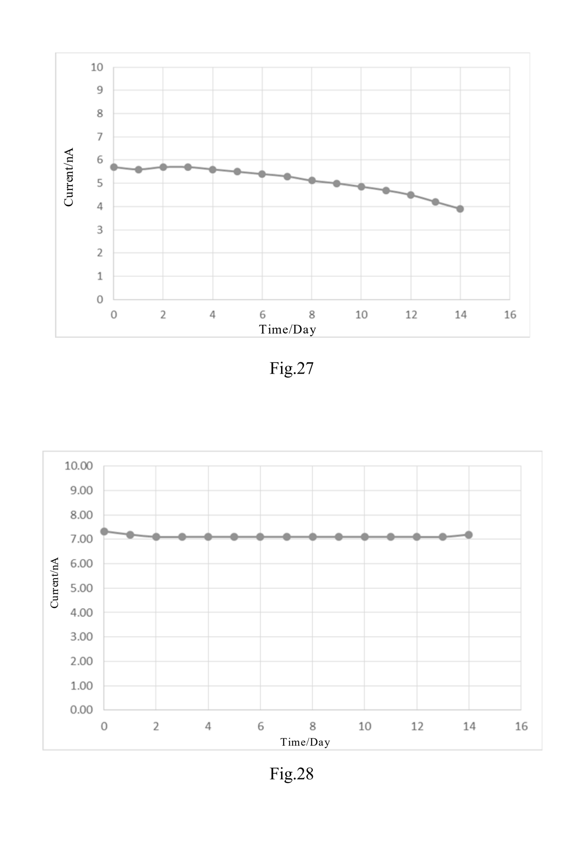

At 37°C, the ketone biosensor was immersed in 3 mM sodium β-hydroxybutyrate (PBS buffer with pH 7.4 as the solvent) and continuously measured for 14 days. Figure 26 shows the response current - test concentration curve of the stability test of Embodiment 1 of the blood ketone sensor involved in the present disclosure. Figure 27 shows the response current - test concentration curve of the stability test of Comparative Embodiment 1 of the blood ketone sensor involved in the present disclosure. Figure 28 shows the response current - test concentration curve of the stability test of Comparative Embodiment 2 of the blood ketone sensor involved in the present disclosure.

As shown in Figures 26-28, the control Embodiment 1 could not maintain stability for 14 days at 37°C, with performance decline starting from the 5th day. However, both Embodiment 1 and control Embodiment 2 could maintain stability for 14 days, as the enzyme sensing layer in them was added with a stabilizer, achieving longer stability for the ketone sensor. The response value of Embodiment 1 was higher than that of control Embodiment 2, indicating that using HEPES buffer as the solvent for the sensing layer reagent is superior to using PBS buffer as the solvent.

### Comparative Embodiment 3

Prepare the monitoring probe with the working electrode in the same way as in Embodiment 1 for blood ketone, and set the enzyme sensing layer on the working electrode to obtain the sensor without the semi-permeable membrane.

The sensor in Embodiment 3 will be exposed to a solution containing 1 mM sodium β-hydroxybutyrate standard PBS buffer (pH 7.4) at 37°C for 1 hour.

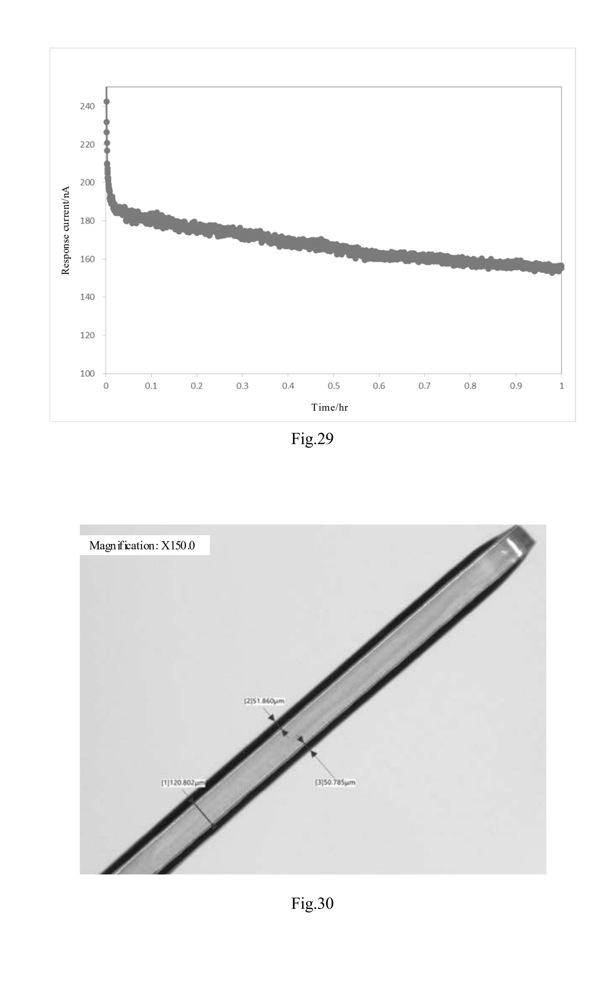

Figure 29 is a response current - test concentration graph of the linear test of Comparative Embodiment 3 of the blood ketone sensor involved in the present disclosure. As shown in Figure 29, within the 1-hour test period, the current signal generated by the sensor gradually decreased from 240 nA to below 155 nA, indicating poor stability. The test results demonstrate that the sensor without a semi-permeable membrane shows poor stability when testing the concentration of sodium β-hydroxybutyrate solution.

### Comparative Embodiment 4

Prepare the monitoring probe with the working electrode in the same way as the proportion 4 of blood ketone, and set the enzyme sensing layer on the working electrode to obtain the sensor without the semi-permeable membrane.

Prepare an 80% ethanol aqueous solution. Configure the corresponding concentration solutions of poly(4-vinylpyridine)-co-polystyrene and PEGDGE as per the table below to obtain the inner membrane solution. Coat the working electrode with the enzyme sensing layer as prepared with the inner membrane solution to make a working electrode with a poly(4-vinylpyridine)-b-polystyrene membrane layer of approximately 35 micrometers.

**Table 12. Composition of Inner Membrane Solution**

| Substance | Concentration (mg/mL) |
|---|---|
| Poly4 - vinylpyridine -b-polystyrene | 70 |
| PEGDGE | 10 |

Prepare an 80% ethanol aqueous solution. Configure the corresponding concentration solutions of (Poly4 - vinylpyridine -g- polyethylene glycol) - co - polystyrene and PEGDGE as per Table 13 to obtain the outer membrane solution. Coat the working electrode with the inner layer membrane with the outer membrane solution to make a working electrode with an outer membrane of approximately 15 microns and a total membrane thickness of 50 microns.

**Table 13. Composition of Outer Membrane Solution**

| Substance | Concentration (mg/mL) |
|---|---|
| (Poly4 - vinylpyridine -g-polyethylene glycol) - co - polystyrene | 70 |
| PEGDGE | 10 |

Figure 30 is a picture of the monitoring probe of the sensor of Comparative Embodiment 4 involved in the present disclosure for blood ketone sensors.

Finally, the obtained ketone sensor was subjected to stability testing; that is, the ketone biosensor of Comparative Embodiment 4 was immersed in 3 mM sodium β-hydroxybutyrate (PBS buffer with pH 7.4 as the solvent) at 37°C and continuously measured for 3 days.

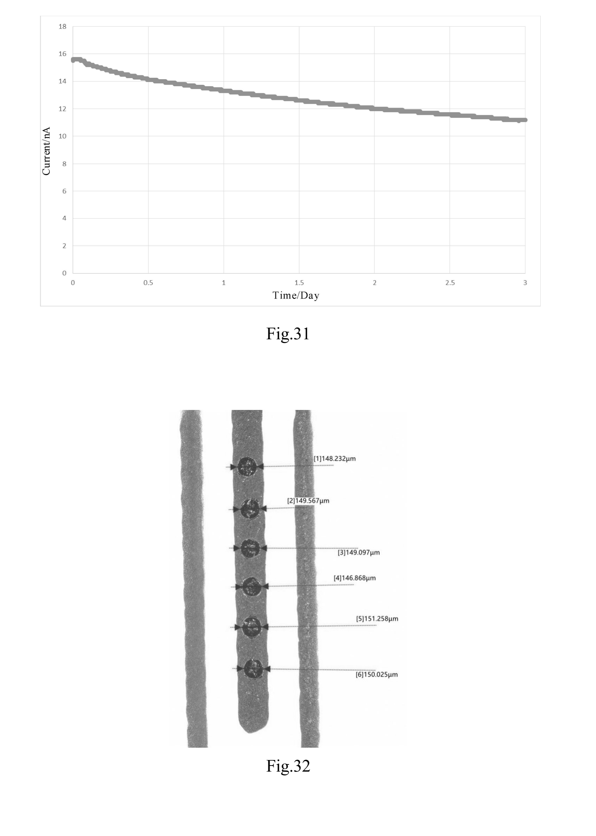

Figure 31 shows the response current - test time curve of the sensor of Comparative Embodiment 4 involved in the present disclosure. As shown in Figure 31, the initial response current of the sensor of Comparative Embodiment 4 was 15.6 nA, and after three days, the sensor response current decreased to 11.2 nA, a total decrease of 28.2%, with an average daily decrease of 9.4% per day. This indicates that the sensor coated only with poly(4-vinylpyridine)-b-polystyrene and (Poly4 - vinylpyridine -g- polyethylene glycol) - co - polystyrene films has poor stability.

The main reason is that compared with the blood ketone implementation Embodiment 1, the membrane layer combination has a higher permeability to sodium β-hydroxybutyrate, indicating that the diffusion limiting effect of this membrane is not strong, unable to reduce the permeability of sodium β-hydroxybutyrate, and the function of restricting the escape of small molecules in the enzyme layer is also poor, resulting in poor stability.

### Comparative Embodiment 5

First, prepare the monitoring probe with the working electrode.

Prepare the corresponding reagents according to the same enzyme layer formula as in Embodiment 1 of blood ketone. Use an ultra-micro pipetting robot to drop-coat the β-hydroxybutyrate-sensing layer solution on the working electrode to form six independent dot patterns, each with a diameter of 150 micrometers; carry out curing to obtain an enzyme sensor with six dot patterns. Figure 32 shows a picture of the working electrode of the blood ketone sensor comparative Embodiment 5 involved in the present disclosure.

Then, the above-mentioned 6-point patterned enzyme sensor was coated with the membrane layer according to the same membrane layer coating scheme as in Embodiment 1 of blood ketone, to obtain the ketone sensor of Comparative Embodiment 5. Finally, the obtained ketone sensor of Comparative Embodiment 5 was subjected to linearity testing: that is, the ketone biosensor was immersed in a standard PBS buffer solution (pH 7.4) at 37°C; the working voltage was applied until the ketone biosensor reached a constant background current, and then 1 mM, 2 mM, 3 mM, 4 mM, 5 mM, 6 mM, 7 mM, and 8 mM concentrations of sodium β-hydroxybutyrate were added to the solution to measure the reaction linearity. After each addition of sodium β-hydroxybutyrate, the solution was continuously stirred during the measurement process to ensure uniform concentration.

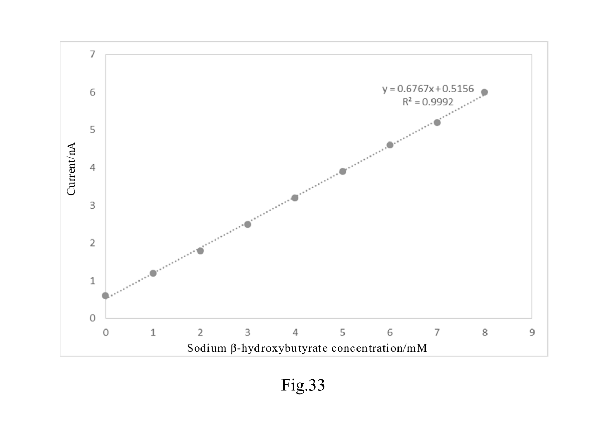

Figure 33 shows the response current - test concentration graph of the linear test of the blood ketone sensor of Comparative Embodiment 5 involved in the present disclosure. As shown in Figure 33, it can be seen that its response to sodium β-hydroxybutyrate shows good linearity in the concentration range of 0-8 mM, but its response sensitivity is relatively low, that is, the slope of the linear fitting curve is 0.677 nA/mM, while the sensitivity of the sensor in Embodiment 1 is 2.08 nA/mM, and the sensitivity of Comparative Embodiment 5 is only 32.5% of that of Embodiment 1. This is because the area of the enzyme layer in Comparative Embodiment 5 is smaller, resulting in a lower reaction area with sodium β-hydroxybutyrate passing through the membrane layer. This indicates that increasing the area of the enzyme layer can effectively increase the sensitivity of the sensor.

In summary, according to the present disclosure, a multi-analyte sensor can be provided, which is capable of simultaneously and continuously monitoring multiple analytes, has a simple manufacturing process, and features high sensitivity and stability.

The various embodiments of the present disclosure have been described in the above specific implementation methods. Although these descriptions directly describe the above embodiments, it should be understood that those skilled in the art can think of modifications and/or variations to the specific embodiments shown and described here. Any such modifications or variations that fall within the scope of this specification are also intended to be included. Unless otherwise specified, the inventor's intention is that the terms in the specification and claims be given the ordinary and customary meanings as understood by those skilled in the art.

## Claims

1. A multi-analyte monitoring sensor, **characterized in that**,
The sensor comprises a working electrode, an enzyme sensing layer disposed on the working electrode, and a polymer membrane layer.
The working electrode comprises a first working electrode and a second working electrode.
The enzyme sensing layer comprises a first analyte enzyme layer and a second analyte enzyme layer. The first analyte enzyme layer is set on the first working electrode, and the second analyte enzyme layer is set on the second working electrode. The second analyte is different from the first analyte.
The polymer membrane layer can be permeable to the first analyte and the second analyte and cover the first analyte enzyme layer and the second analyte enzyme layer.

2. The multi-analyte monitoring sensor according to claim 1, **characterized in that**,
The first analyte mentioned is glucose.

3. The multi-analyte monitoring sensor according to claim 1 or 2, **characterized in that**,
The second analyte is any one of β-hydroxybutyrate, lactic acid, ethanol, uric acid, urea, acetylcholine, amylase, bilirubin, cholesterol, chorionic gonadotropin, creatine kinase, creatine, creatinine, DNA, fructosamine, glutamine, hormone, ketones, lactate, oxygen, peroxide, prostate-specific antigen, prothrombin, RNA, troponin, digoxin, digitonin, theophylline, warfarin or antibiotic.

4. The multi-analyte monitoring sensor according to claim 1 or 2, **characterized in that**,
The polymer membrane layer comprises a film polymer, which is a vinylpyridine-based polymer, polyurethane, sodium polystyrene sulfonate, poly(N-isopropylacrylamide), polyacrylic acid, polyvinyl alcohol, polyvinylpyrrolidone, poly(oligo(ethylene glycol) methyl ether methacrylate), or a block copolymer or blend of the above polymers.

5. The multi-analyte monitoring sensor according to claim 4, **characterized in that**,
The polymer membrane layer also includes a crosslinker and a regulator, and the mass fraction of the film polymer in the polymer membrane layer is 80% to 100%.

6. The multi-analyte monitoring sensor according to claim 1 or 2, **characterized in that**,
The polymer membrane layer has biocompatibility.

7. The multi-analyte monitoring sensor according to claim 6, **characterized in that**,
The polymer membrane layer comprises a first membrane layer that is biocompatible and has a diffusion-limiting effect on the first analyte and the second analyte.

8. The multi-analyte monitoring sensor according to claim 6, **characterized in that**,
The polymer membrane layer comprises a first membrane layer that has a diffusion-limiting effect on the first analyte and the second analyte, and a second membrane layer that is biocompatible. The second membrane layer is further away from the enzyme sensing layer than the first membrane layer.

9. The multi-analyte monitoring sensor according to claim 1, **characterized in that**,
The sensitivity of the first working electrode differs from that of the second working electrode by no more than six times.

10. The multi-analyte monitoring sensor according to claim 1 or 9, **characterized in that**,
The area of the first analyte enzyme layer is greater than or less than the area of the second analyte enzyme layer.

11. The multi-analyte monitoring sensor according to claim 1 or 9, **characterized in that**,
The polymer membrane layer comprises a negatively charged membrane layer that has a diffusion-limiting effect on the first analyte and the second analyte.

12. The multi-analyte monitoring sensor according to claim 3, **characterized in that**,
The second analyte is β-hydroxybutyrate. The second analyte enzyme layer comprises β-hydroxybutyrate dehydrogenase, diaphorase, coenzyme, and electron mediator. In the second analyte enzyme layer, the mass fraction of β-hydroxybutyrate dehydrogenase is 10% to 35%, the mass fraction of diaphorase is 5% to 40%, the mass fraction of coenzyme is 10% to 45%, and the mass fraction of the electron mediator is 10% to 50%.

13. The multi-analyte monitoring sensor as claimed in claim 1, **characterized in that**,
The sensor also includes a substrate, and the working electrode is set on the substrate.

14. The multi-analyte monitoring sensor according to claim 13, **characterized in that**,
The sensor also includes a reference electrode and a counter electrode. The first working electrode and the second working electrode are respectively located on both sides of the substrate, and the reference electrode and the counter electrode are respectively located on both sides of the substrate.
